# EUROPEAN PATENT APPLICATION

(11) **EP 3 730 625 A1**
(43) Date of publication of application: **28.10.2020**
(21) Application number: 19000200.6
(22) Date of filing: 24.04.2019
(51) Int. Cl.: C12Q 1/6806, C12Q 1/6869

(54) **USE OF DROPLET SINGLE CELL EPIGENOME PROFILING FOR PATENT STRATIFICATION**

(71) Applicant: HiFiBiO SAS, 75014 Paris (FR); Institut Curie, 75005 Paris (FR); PSL, 75005 Paris (FR)
(72) Inventor: Gérard, Annabelle Patricia Veronique, 91120 Palaiseau (FR); Grosselin, Kevin, 75014 Paris (FR); Griffiths, Andrew David, 75006 Paris (FR); Vallot, Céline Victoire Delphine Marie, 75017 Paris (FR)
(74) Representative: CH Kilger Anwaltspartnerschaft mbB

(57) **Abstract**

An aspect of the invention relates to a method for the diagnosis and/or prognosis of drug resistance, wherein single cell chromatin states are profiled in cells obtained from a subject by using a microfluidic system.

## Description

### FIELD OF THE INVENTION

The present invention is in the field of molecular biology, drug resistance and microfluidics. Particularly, the invention relates to a method of assaying nucleic acid in microfluidic droplets for the diagnosis and/or prognosis of drug resistance and patient stratification.

### BACKGROUND

Eukaryotic genomes are organized into chromatin which enables not only to compact DNA but also regulates DNA metabolism (replication, transcription, repair, recombination). A current challenge is thus to understand (i) how functional chromatin domains are established in the nucleus, (ii) how chromatin structure/information is dynamic through assembly, disassembly, modifications and remodeling mechanisms and (iii) how these events participate/maintain in disease establishment, progression and relapse of disease. Understanding these events will allow identifications of novel mechanisms of disease progression and new therapeutics targets, as well as controlling effect of therapeutics molecules.

In addition, a fundamental research question in biology is to understand how hundreds of distinct cell types arise from identical genetic material in multicellular organisms. The many different cell types can't be explained solely by genetics but rather by an additional information that can bridge phenotype to genotype. In 1942, Conrad H. Waddington coined the term Epigenetics as "the branch of biology which studies the causal interactions between genes and their products, which bring the phenotype into being". This additional layer of "epigenetic information" is stored in the form of chemical modifications to both DNA and histone proteins that constitute the chromatin. Epigenetic mechanisms through chromatin modifications regulate gene expression and shape specific chromatin landscapes, which allow predictions to be made about cell type and tissue identity. DNA and histone modifications are involved in various DNA-based processes by serving as recognition sites for effector proteins capable of reading information and by stabilizing their binding to the chromatin. The large abundance of histone modifications enables a tight control of chromatin structure and a great flexibility in the regulation of DNA-based processes. This diversity leads to crosstalk between histones that can be modified at different sites simultaneously (Wang et al. 2008, Nature Genetics 40(7):897-903).

Histone modifications can positively or negatively affect each other. In addition, communication between histone modifications also exists with other chromatin modifications such as DNA methylation, which all participate to fine-tune the overall regulation of the biological functions (Du et al. 2015, Nature Reviews Molecular Cell Biology, 16(9):519-532).

DNA and histone modifications contribute to the definition of epigenomic signatures within distinct chromatin states, which are highly indicative of cell type and tissue identity. The genome-wide profiling of these marks can be leveraged to understand the global landscape of genome regulation and then, for example, distinguish epigenomic differences in the context of normal and disease cell states (Consortium Epigenomics 2015, Nature 518(7539):317-329). However, the current state of the chromatin profiling technologies does not allow studying cellular heterogeneity nor detect cell-to-cell variation in chromatin states.

Genome-wide mapping of epigenetic modifications, epigenetic markers/erasers, factors playing a role in chromatin structure, organization in 2D and 3D using traditional ChlP-seq method requires a large number of cells to generate high quality binding site profiles. Several studies have shown optimized ChlP-seq protocols to reduce input material from millions to hundreds of cells without losing resolution in the detection of enriched or depleted regions (Adli et al. 2010, Nature Methods 7(8):615-618; Brind'Amour et al. 2015, Nature Communications 6:6033; Ma et al. 2018, Science Advances 4(4):eaar8187)., However, these methods only yield an averaged snapshot of the modification status, without providing insight into the epigenetic heterogeneity.

Profiling histone modifications at single-cell resolution remains challenging, in part because the level of noise associated with non-specific binding during the immunoprecipitation tends to increase with low quantity of starting material. Immunoprecipitating chromatin from one single cell is technically feasible but would lead to highly variable results.

Chromatin from isolated single cells can be indexed beforehand with a specific and unique DNA sequence (barcode), and then combined with indexed chromatin from a couple to several thousands of cells to perform immunoprecipitation in bulk as in traditional ChlP-seq protocol. This method circumvents the issue associated with high experimental noise in the immunoprecipitation of low input material, while retaining the single-cell information. Indeed, barcodes being specific of one cell, each read can be attributed to its originating cell after sequencing. However, like other single-cell technologies in which molecular indexing is involved, only indexed nucleosomes have the potential to be amplified and sequenced.

In this regard, Rotem developed a Drop-ChIP technology that combines chromatin indexing method with droplet-based microfluidics to profile histone modifications of thousands of cells (Rotem et al. 2015, Nature Biotechnol. 33(11):1165-1172). The droplet format provides a versatile tool for performing single-cells assays. Following the steps of compartmentalization, lysis and chromatin fragmentation by microccocal nuclease of the cell in droplets, said droplets are then merged one-to-one with a second population of droplets containing DNA barcodes, allowing chromatin indexing at the single-cell level.

Although Drop-ChiP was used to reveal distinct chromatin states within a population of embryonic stem cells, the single-cell information was limited to as few as hundreds of unique enriched loci detected per cell due to low chromatin indexing efficacy or poor recovery of indexed nucleosomes. Notably, Drop-ChIP technology suffers from two major limitations, which may negatively impact the amount of information recovered per cell. Firstly, only symmetrically indexed nucleosomes can be amplified and can be part of the sequencing library. This requirement dramatically increases the stringency of the system and imposes a strong selection on the nucleosomes (i.e. only those with both ends ligated to a barcode). Secondly, amplification of indexed nucleosomes only relies on numerous cycles of Polymerase Chain Reaction (PCR), which increases the probability to introduce amplification bias and errors.

Spontaneous heterogeneity of chromatin states in untreated cells may be a key molecular component in the acquisition of drug-resistance, regardless of the mechanism of action of the cancer treatment. Many types of cancer are initially susceptible to chemotherapeutics and over the time can develop resistance through these and other mechanisms. However, methods of drug resistance can be disease-specific while others can be can evolutionarily conserved. The emergence of resistance to chemotherapy and targeted therapies is a major challenge for the treatment of cancer. The genetic heterogeneity within untreated tumors is now considered to be a key determinant of resistance. In addition, non-genetic and particularly transcriptional and epigenetic mechanisms are anticipated to play a role in the adaptation of cancer cells confronted with environmental, metabolic or therapy-related stresses (Rathert, P. et al. Nature 525, 543-547, (2015); Kim, C. et al. Cell 173, 879-893 e813, (2018).). Modulation of chromatin structure *via* histone modification is a major epigenetic mechanism and regulator of gene expression, however, the contribution of chromatin features to tumor heterogeneity and evolution remains unknown. In view of the above limitations affecting methods known in the art, it is evident that an improved method and compositions for the diagnosis and/or prognosis of drug-resistance associated with distinct chromatin states using single-cell epigenetic profiling in microfluidic droplets is required.

Certain embodiments of the invention disclose that rare populations of cells in untreated, drug-sensitive tumors display chromatin features that match those of resistant cells. The inventors have developed a droplet microfluidics approach to profile chromatin landscapes of thousands of cells at single-cell resolution with a coverage of up to 10,000 loci/cell, which approach has a variety of applications as disclosed herein.

### SUMMARY

An aspect of the invention relates to compositions and methods for the diagnosis and/or prognosis of drug resistance, wherein single cell chromatin states are profiled in cells obtained from a subject by using a microfluidic system, the method comprising the steps of:
a. providing at least a droplet of first type, wherein said droplet of first type comprises
   i. a biological element,
   ii. a lysis buffer, and
   iii. a nuclease,
b. collecting said droplet of first type under conditions that temporarily inactivate said nuclease,
c. incubating said droplet of first type, thereby reactivating said nuclease,
d. providing at least a droplet of second type, wherein said droplet of second type comprises a nucleic acid sequence,
e. merging said droplets of first and second type, thereby generating a droplet of third type,
f. incubating said droplet of third type, thereby linking said nucleic acid sequence to one or more genomic region(s) of interest,
g. sequencing said one or more genomic region(s) of interest.

The one or more genomic region of interest is a modified genomic region, which comprises a nucleic acid sequence and/or a protein complex associated with a nucleic acid sequence.

The modified genomic region comprises a post-translational modification selected from the group comprising acetylation, amidation, deamidation, carboxylation, disulfide bond, formylation, glycosylation, hydroxylation, methylation, myristoylation, nitrosylation, phosphorylation, prenylation, ribosylation, sulphation, sumoylation, ubiquitination and derivatives thereof, preferably a methylation.

According to the invention, the cells derive from a subject in a diseased state and/or suspected to be in a diseased state or in a healthy subject. The subject is in a diseased state, and the diseased state comprises cancer, infectious disease, auto-immune disease, metabolic disease, inflammation disease, genetic and non-genetic diseases.

The method according to the invention, wherein the diseased state is breast cancer.

The method according to the invention, wherein the cells are untreated and/or treated. The treated cells are treated with a therapeutic agent, or a chemotherapeutic agent, or a chemical drug, or biologic drugs.

The method according to the invention, wherein the treated cells are treated with Tamoxifen and Capecitabine.

The method according to the invention, wherein the single cell chromatin state has lost chromatin marks for genes that promote drug resistance, wherein the chromatin marks are histone modifications H3K4me3 and H3K27me3.

### BRIEF DESCRIPTION OF THE DRAWINGS

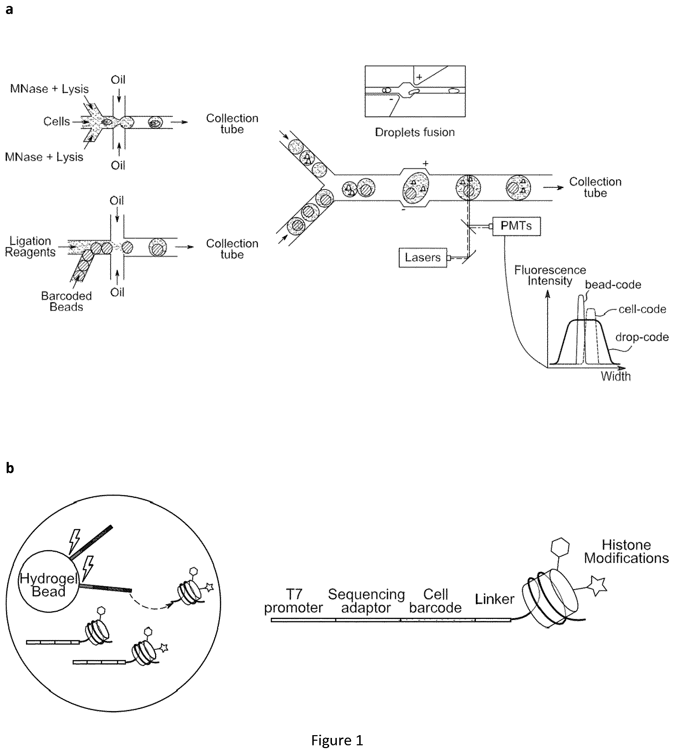

Figure 1 depicts the microfluidic workflow according to the invention. (A) Cells are compartmentalized in 45pl droplets with the reagents necessary for lysis and chromatin fragmentation. In parallel, hydrogel beads carrying DNA barcodes are encapsulated in 100pl droplets with ligation reagents. The two emulsions are reinjected in a fusion device, the barcode drops (100pl) and the nucleosome drops (45pl) are paired asymmetrically and an electric field triggers the fusion. Fused droplets are scanned one-by-one by a laser beam and the composition of each droplet is analyzed in real time. (B) Emulsion of fused droplets is collected for nucleosomes barcoding in droplets. Barcodes are released from the beads by photo-cleavage and ligated to the nucleosomes. The content of droplets is combined, immunoprecipitated and the enriched DNA is sequenced. The deconvolution of the barcode associated reads attributes all sequences to their originating cell to reconstruct single-cell chromatin profile.

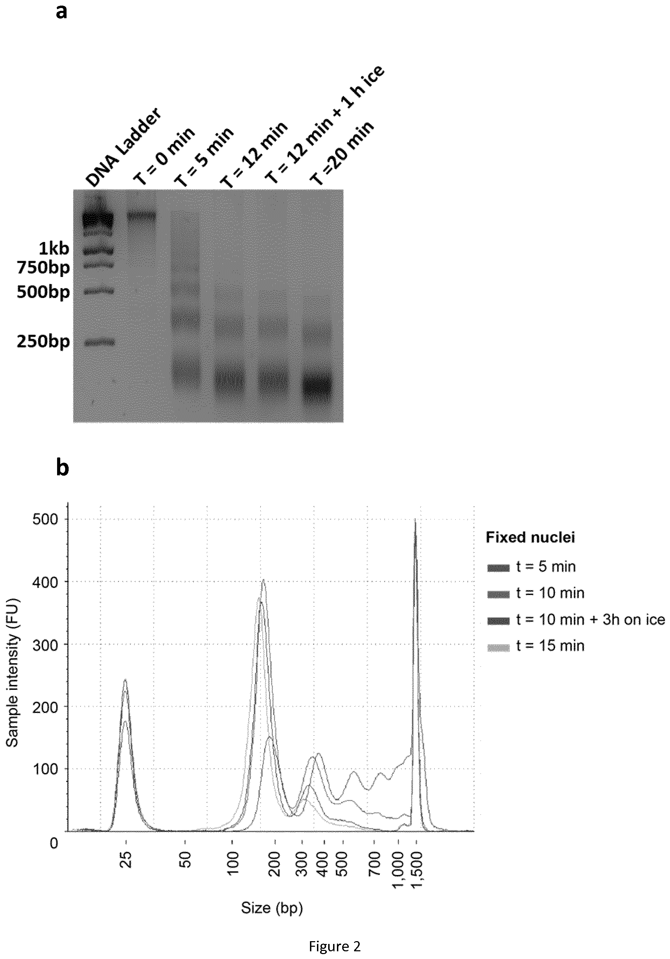

Figure 2 depicts the synchronization and pause of the MNase activity between droplets. Particularly, Figure 2a shows gel electrophoresis of DNA fragments from human Jurkat T cell at different time point of MNase incubation. A t = 0min, DNA is not fragmented yet, confirming that MNase activity is synchronized upon droplets collection on ice. Time point t = 12min + 1h ice shows similar digestion profile as after 12min of incubation, confirming that MNase activity is paused when droplets are stored on ice. Figure 2b depicts the pause of MNase activity in droplets starting from fixed nuclei. MNase is paused for 3h without chromatin over-digestion.

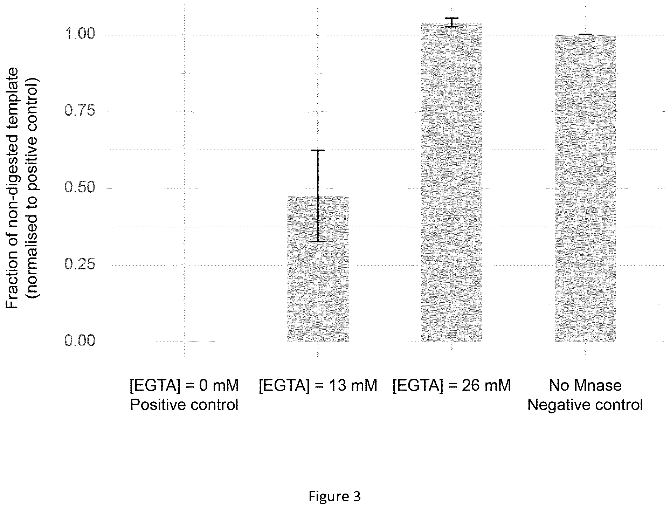

Figure 3 depicts the concentration of EGTA necessary to fully inactivates MNase activity in droplets. The fraction of oligonucleotide remaining after incubation was measured by TapeStation and normalized to the digestion negative control (i.e droplets without MNase). 26 mM final concentration of EGTA fully inactivated MNase in droplets. Barplot shows the mean fraction of non-digested oligonucleotides for duplicates and the error bars correspond to the standard deviation.

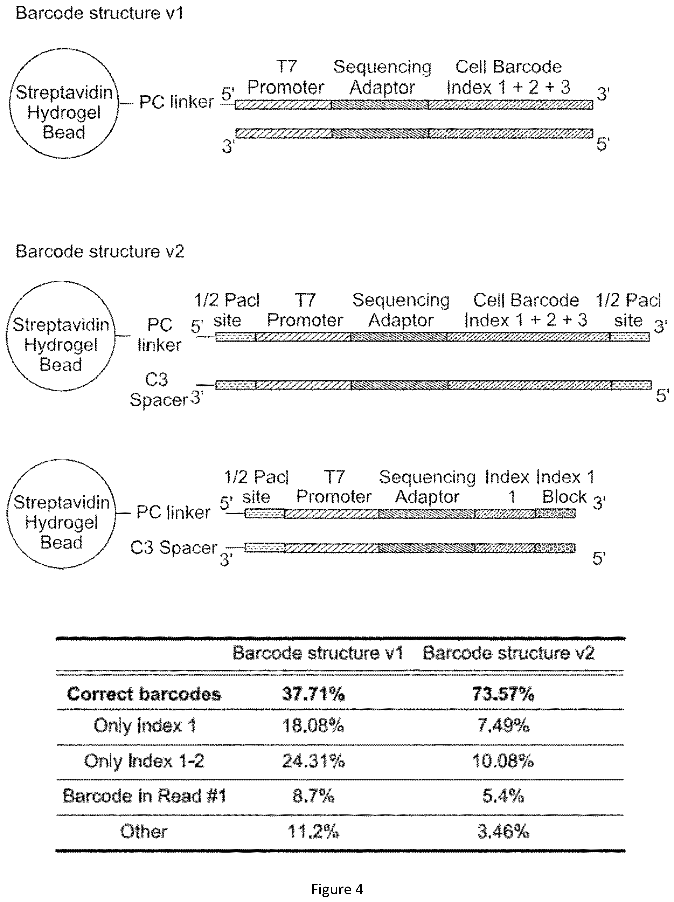

Figure 4 depicts the nucleic acid sequence according to the invention. The new structure (v2) allows digestion of barcode concatemers by addition of one half of Pacl restriction site on both ends of the barcodes. A C3-spacer at the 3'-end of the bottom strand is also added to enforce the direction of the ligation. Non-full-length barcodes are completed with a protecting group containing modified bases modifications that prevent unwanted ligation. The table depicts the proportion of correct barcodes identified post-sequencing with each of the barcode structure.

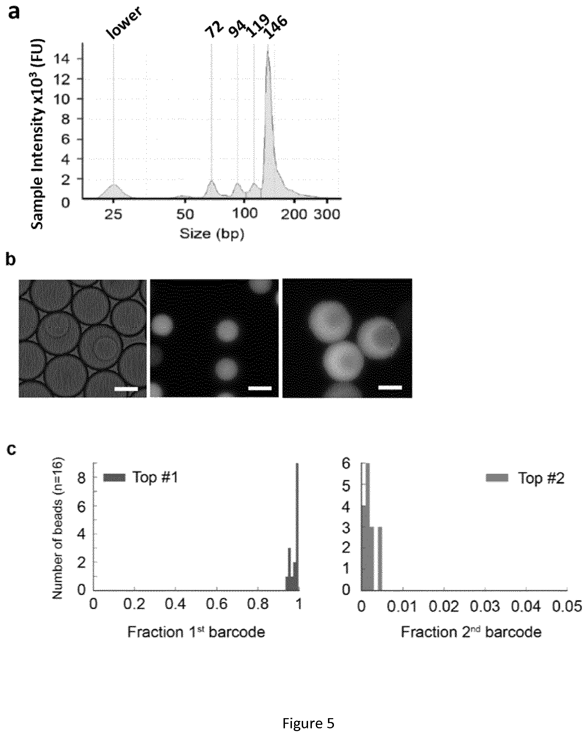

Figure 5 depicts the quality control of barcoded hydrogel beads. (a) Tapestation profile of DNA barcodes after photo-cleavage from hydrogel beads shows the presence of full-length barcodes (larger peak at 146 bp), as well as intermediates that failed to be completed (peaks at 72 bp, 94 bp and 119 bp). (b) Imaging of hydrogel beads in droplets using epifluorescence microscopy. From left to right: (i) bright field image; (ii) imaging after hybridization of a DNA probe complementary to the Illumina sequencing adaptor onto the barcodes; (iii) same as (ii) after release of the barcodes in droplets by photo-cleavage. Scale bars are 35 µm. (c) Single bead deep sequencing results showing the fraction of the first-two most abundant barcodes of 16 beads. In average, 97.7% of the barcodes present on a bead match to the same sequence while the second most abundant barcode represents only 0.17% of all sequencing reads.

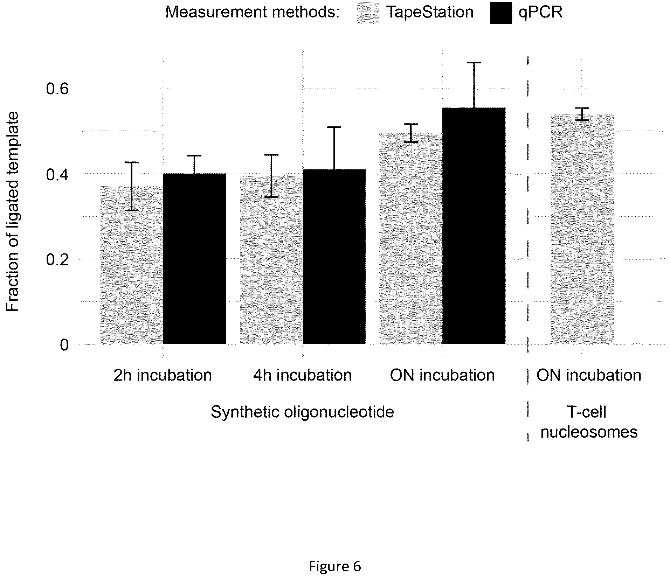

Figure 6 depicts the estimation of the ligation efficiency in droplets. The ligation was performed for 2h, 4h and overnight incubation. Both measurement methods gave similar estimation of the fraction of ligated product and a significant increase in efficiency was observed after an overnight incubation (^{∼}10%). Barplot shows the mean fraction of ligated oligonucleotides for duplicates (experiments performed on two different days) and the error bars correspond to the standard deviation.

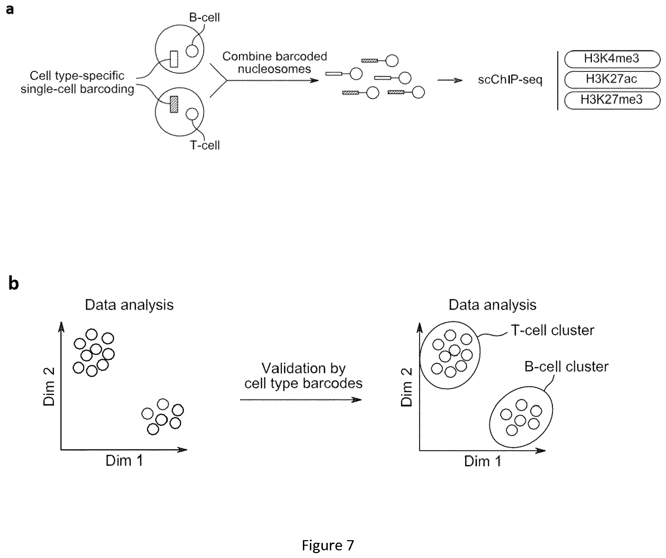

Figure 7 depicts the proof of concept study and expected outcomes. (a) Human B and T lymphocytes were separately encapsulated and specifically indexed in droplets. Indexed chromatin from the two emulsions were then combined for ChlP-seq targeting 3 distinct histone modifications (H3K4me3, H3K27ac and H3K27me3). (b) Data analysis would group cells into two clusters using an unsupervised clustering approach. The correct clustering of B and T-cells based on their chromatin profiles would then be confirmed by the cell type-specific barcode sequence.

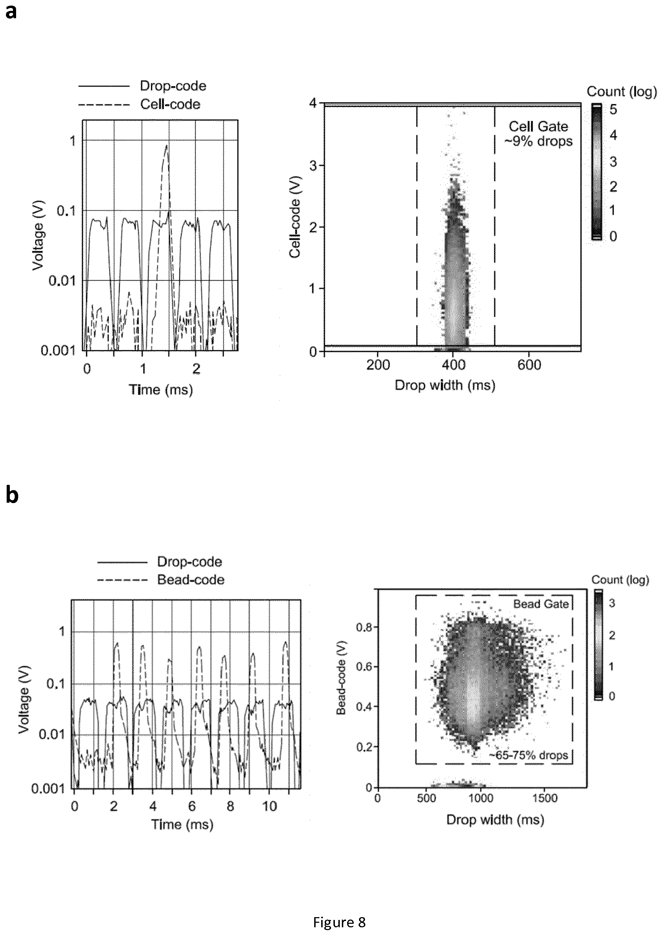

Figure 8 depicts monitoring of cells and hydrogel encapsulation in droplets. (a) Experimental time trace recorded for droplets analyzed at 1.8 kHz. Orange fluorescence is present in all droplets and used to control for the size of the drops (drop-code). Green fluorescence indicates the presence of a cell in a droplet (cell-code). Plot of cell-code intensity (green) vs drop-code intensity (orange) in each droplet. Droplets containing a cell have a high green cell-code intensity allowing counting of encapsulated cells with the definition of a cell-gate above the noise level. A cell density adjusted to λ = 0.1 results in ^{∼}9% of the droplets containing one single cell. (b) Experimental time trace recorded for 100 pi droplets analyzed at 650 Hz. Orange fluorescence is present in all droplets and used as a drop-code to control for the size of the droplets. Red fluorescence indicates the presence of a hydrogel bead in a droplet (bead-code). Plot of bead-code intensity (red) versus drop-code intensity (orange) in each droplet. By using a close-packed ordering of the beads, 65% to 75% of the droplets contain one bead.

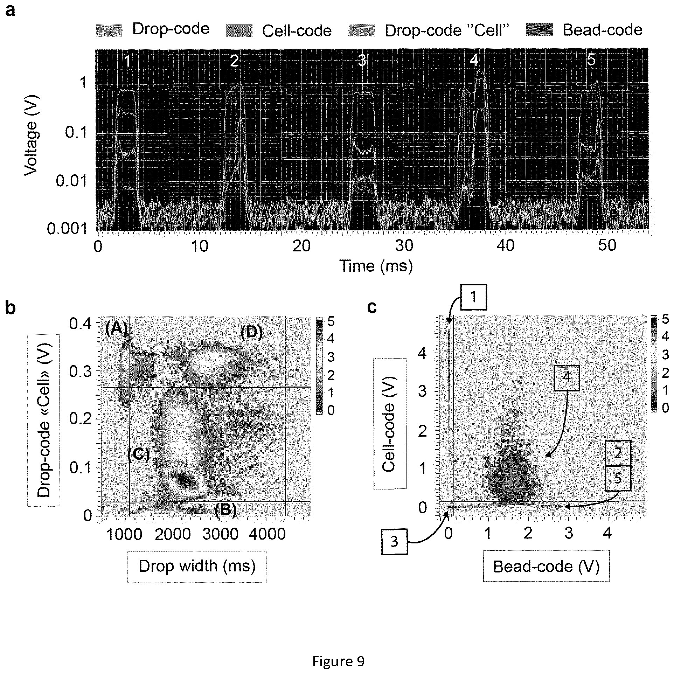

Figure 9 depicts live monitoring of droplets fusion. (a) Experimental time trace recorded for droplets after fusion at 150 Hz. Orange fluorescence is present in all droplets and used as a drop-code to control for the size of the fused drops. Green fluorescence indicates the presence of a cell and the red fluorescence indicates the presence of a hydrogel bead. Blue fluorescence is a drop-code specific of the cell emulsion. (b) Plot of drop-code "cell" intensity versus drop-code intensity in each droplet defining 4 main populations of droplets after fusion. The main population in the middle represents droplets correctly paired and fused (70% to 80%). Unpaired droplets from the bead-emulsion are in the bottom population and unpaired droplets from the cell-emulsion with a high blue-fluorescence intensity are in the top-left population. The last population (top right) is associated with incorrectly-paired droplets containing 2 cell-droplets fused with one bead-droplet. (c) Plotting cell-code intensity versus bead-code intensity in each droplet enables precise counting of useable drops (those containing one cell and one bead). Droplets from the time trace in panel (a) are indicated as example of the different populations.

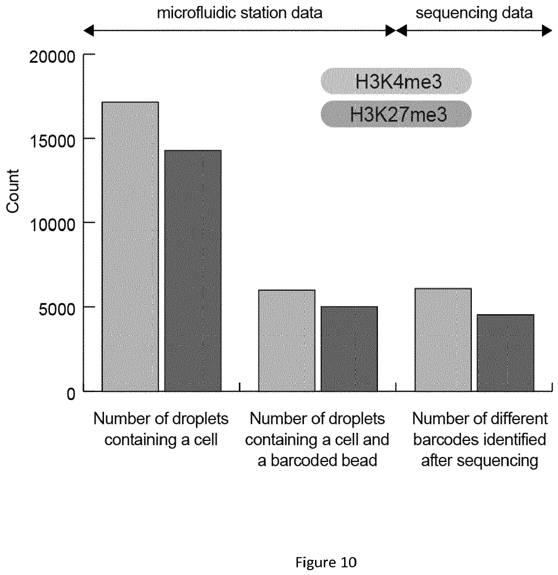

Figure 10 depicts the total number of cells and the number of cells co-encapsulated with a barcoded hydrogel bead detected by fluorescence on the microfluidic station in H3K4me3 and H3K27me3 single-cell ChlP-seq experiments. Analysis of sequencing data showed a number of identified barcodes closely related to the number of droplets containing both a cell and a bead counted on the microfluidic station, suggesting high overall efficacy of the system.

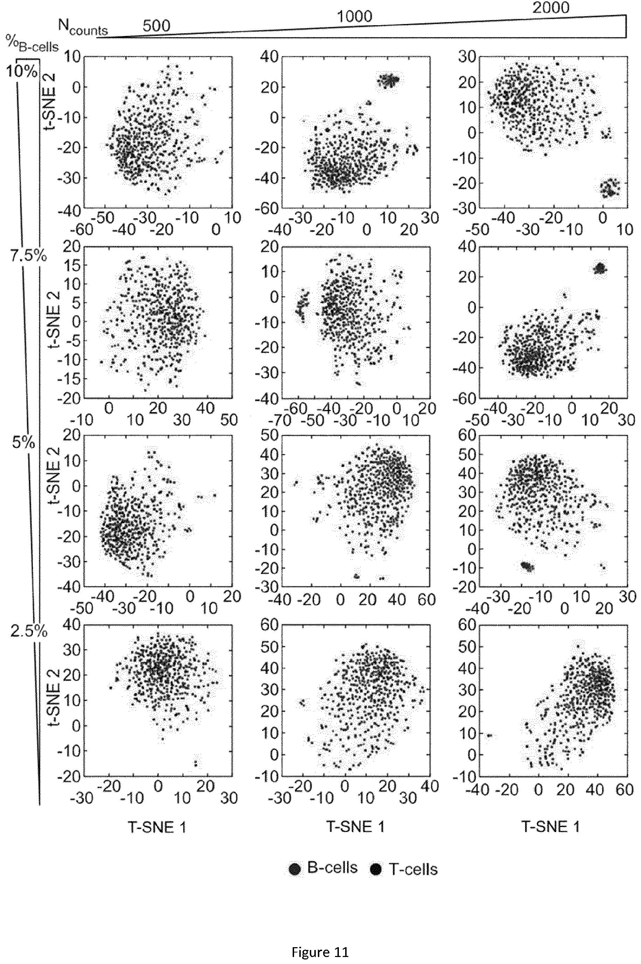

Figure 11 depicts the sensitivity of the scChlP-seq procedure to identify subpopulations. t-SNE plots representing H3K27me3 scChlP-seq dataset in in-silico simulation of detection limit with varying proportion of spiked-in B-cells in T-cells population (from top to bottom) and varying threshold of uniquely mapped reads per barcode (from left to right). Points are colored according to cell type specific barcode sequence.

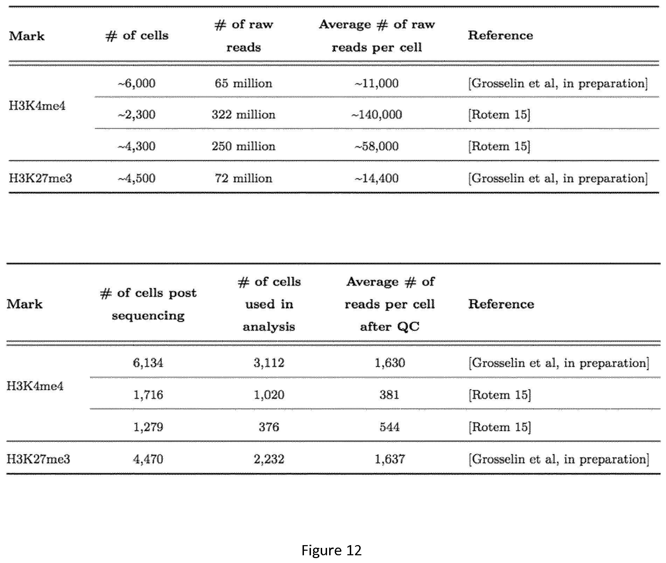

Figure 12 depicts sequencing performance in Drop-ChIP versus inventors' procedure. Table 1 compares the number of expected cells per sequencing library, the number of raw sequencing reads as well as the average number of raw reads per cell in Drop ChIP and in inventors' scChlP-seq system. Table 2 compares the number of cells identified after sequencing, the final number of cells used in analysis after QC and the average number of useable reads per cell after QC in Drop ChIP and in inventors' scChlP-seq system.

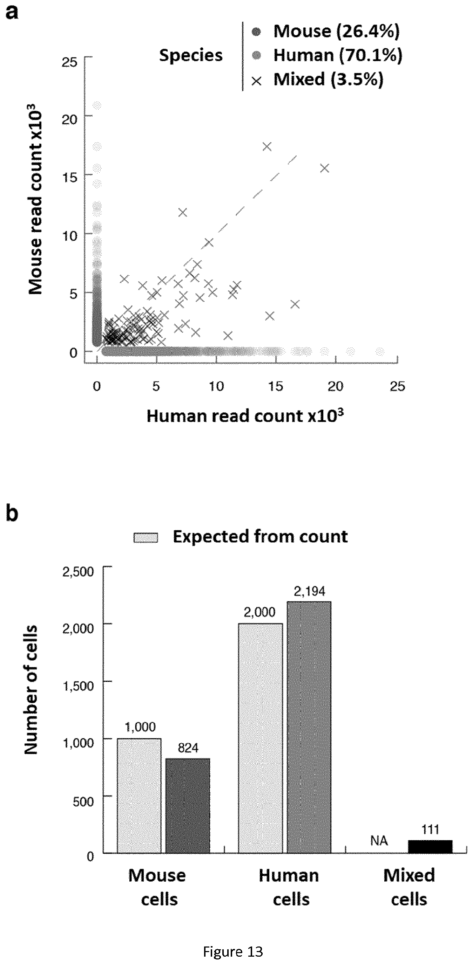

Figure 13 depicts human and mouse cells mixture confirms single-cell resolution. (a) Scatter plot of number of reads per barcode aligning to the mouse versus human reference genome showing that 96.5% of the barcodes are specific to one species (at least 95% of the reads with the same barcode mapping to one of the two species). The percentage of mouse (26.4%), human (70.1%) and mixed (3.5%) species was close to expected values based on a Poisson distribution of cells in droplets with an average number of cells per droplets λ of 0.1 (32.6%, 65.2% and 2.2%, respectively). (b) Barplot showing the number of barcodes (corresponding to the number of cells) identified for each species (blue, red and black bars) in comparison to the expected number of cells counted on the microfluidic station (gray bars; 3,000 in total from a mixture comprising 1/3 mouse cells and 2/3 of human cells).

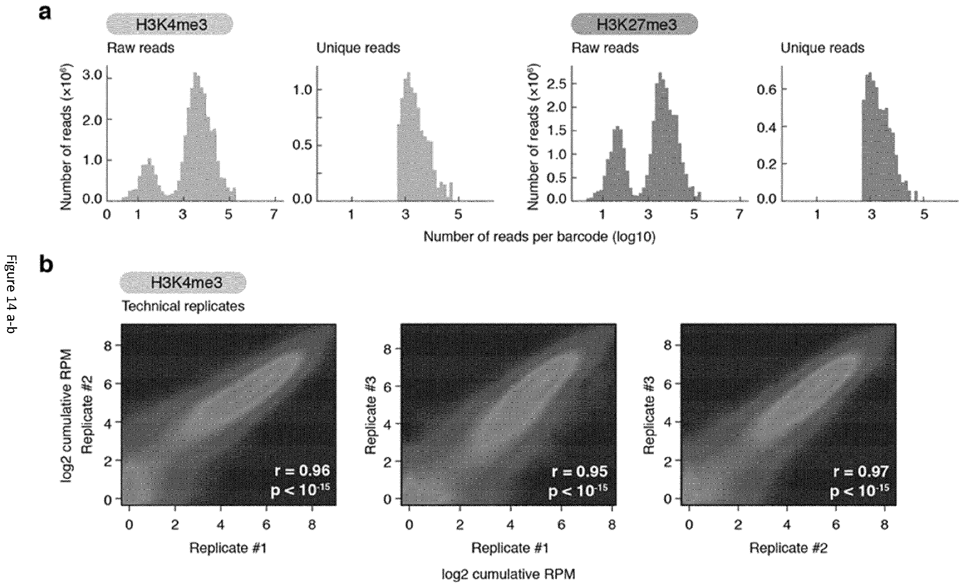

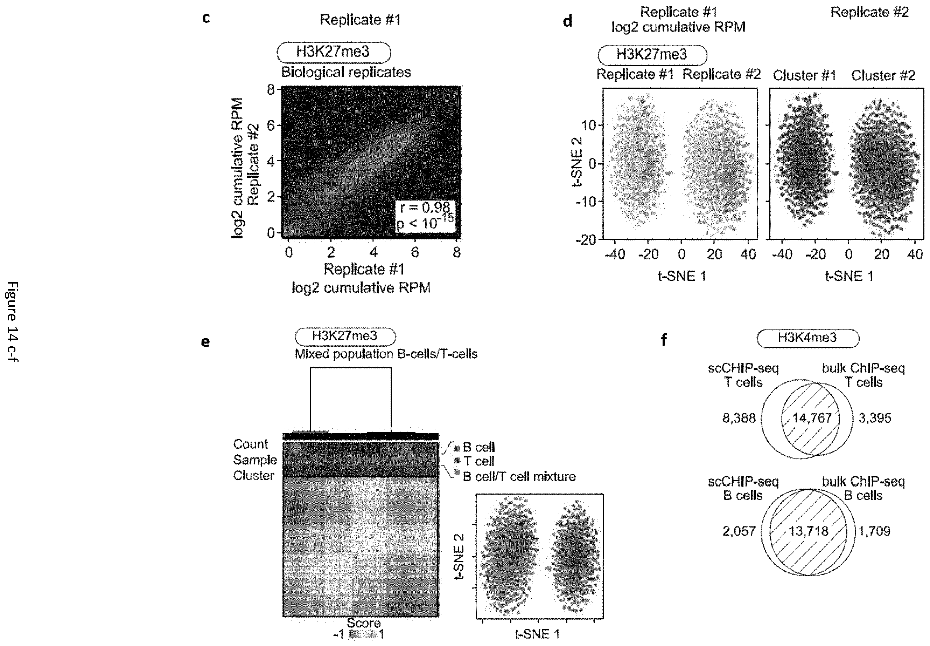

Figure 14 depicts the clustering of single-cell ChlP-seq data reveal cell type-specific biological similarities. (a) Histograms of the distribution of scChlP-seq raw and unique sequencing reads per barcode (i.e. per cell) in H3K4me3 and H3K27me3 single-cell ChlP-seq datasets. (b) Density scatterplots representing log2 cumulative counts between three independent fractions of the same emulsion of B cells collected and processed in parallel to generate H3K4me3 single-cell ChlP-seq datasets. Correlation between replicates is calculated based on the cumulative count per million reads in 5 kb genomic bins across single-cells. Pearson's correlation scores and p-values are computed genome-wide. (c) Density scatter plot for two biological replicates corresponding to two emulsions of B cells collected from different cell culture flasks and processed with different batches of barcoded hydrogel beads to generate H3K27me3 single-cell ChlP-seq datasets. Correlation between replicates is calculated based on the cumulative count per million reads in 50 kb genomic bins across single-cells. Pearson correlation's score and p-value are computed genome-wide. (d) t-SNE plots representing H3K27me3 scChlP-seq data from two biological replicates, colored according to the batch of origin (left) or consensus clustering results (right), arguing against a batch effect of cell population clustering. (e) Left panel: hierarchical clustering and corresponding heatmap of cell-to-cell Pearson correlation scores for H3K27me3 scChlP-seq data from a mixed population of 1:1 human B cells and T cells together with separately barcoded B cells and T cells. The unique read count, the batch of origin and the consensus clustering result is indicated above the heatmap. Right panel: Corresponding t-SNE plot, points from the mixed population are colored in gray, while points from the separately barcoded B cells and T cells are in different grey shadows according to the cell type-specific barcode sequence. (f) Venn diagrams comparing for T and B cells datasets the H3K4me3 peaks detected by single-cell and bulk approaches.

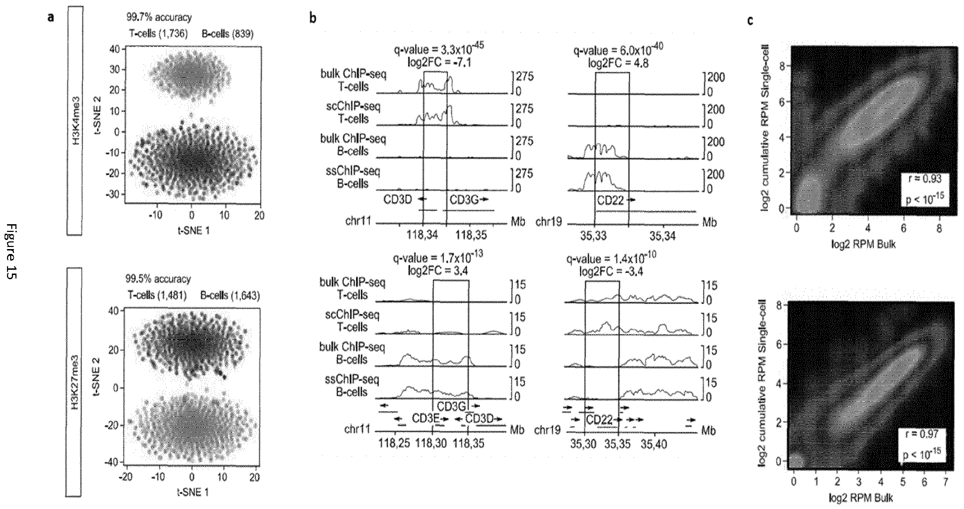

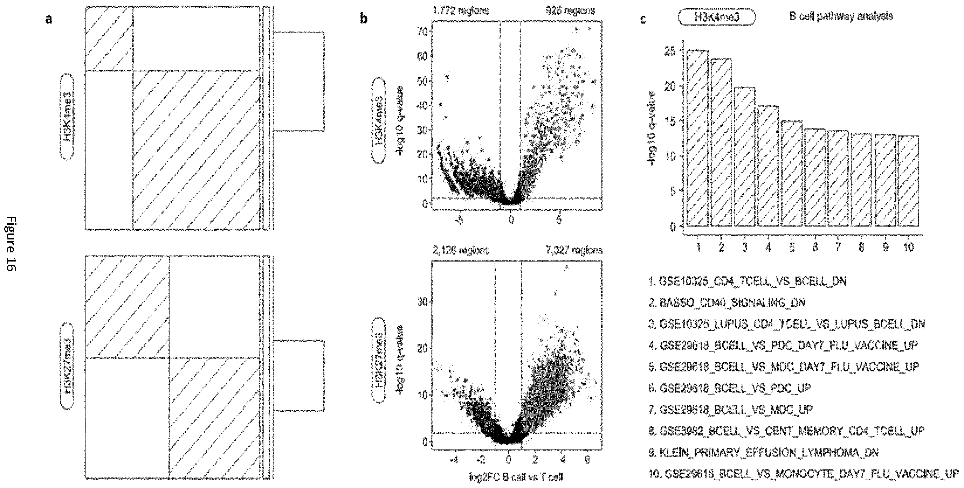

Figure 15 depicts the reconstruction of the cell type specific chromatin states from single-cell ChIP-seq profiles. (a) t-SNE plots representing H3K4me3 and H3K27me3 scChlP-seq data sets from human B and T lymphocytes separately indexed in droplets using hydrogel beads carrying both single-cell and cell-type specific barcodes, mixed for immuno-precipitation. Points are in different grey shadow according to the cell-type specific barcode sequence. Accuracy represents the agreement between the classification by consensus clustering of scChlP-seq data (Fig. 16a) and known cell identity, assessed by the cell-type specific barcodes. (b) Snapshots of differentially enriched loci (Fig. 16b) with cumulative single-cell profiles for each cell type and bulk profiles. Differentially bound regions identified by a Wilcoxon signed-rank test are indicated in gray with the corresponding adjusted p-value and log2 fold change. (c) Scatter plots displaying log2 RPM (read count per million of mapped reads) enrichments in cumulative single-cell versus bulk ChlP-seq data, calculated within 5 kb genomic bins for H3K4me3 and 50 kb for H3K27me3. Pearson correlation scores and p-values are computed genome-wide.

Figure 16 depicts single-cell ChlP-seq data distinguish human T cells (Jurkat) from human B cells (Ramos). (a) Consensus clustering matrix for H3K4me3 (top panel) and H3K27me3 (bottom panel) scChlP-seq datasets. Consensus score ranges from 0 (white: never clustered together) to 1 (dark blue: always clustered together). (b) Volcano plots representing adjusted p-values (Wilcoxon rank test) versus fold-changes for differential analysis comparing chromatin features between B cells and T cells (thresholds of 0.01 for q-value and 1 for |log2FC|) for H3K4me3 (top panel) and H3K27me3 (bottom panel) scChlP-seq datasets. (c) Barplot displaying the -log10 of adjusted p-values from pathway analysis in H3K4me3 scChlP-seq dataset. The top 10 significant gene sets are indicated below the barplot.

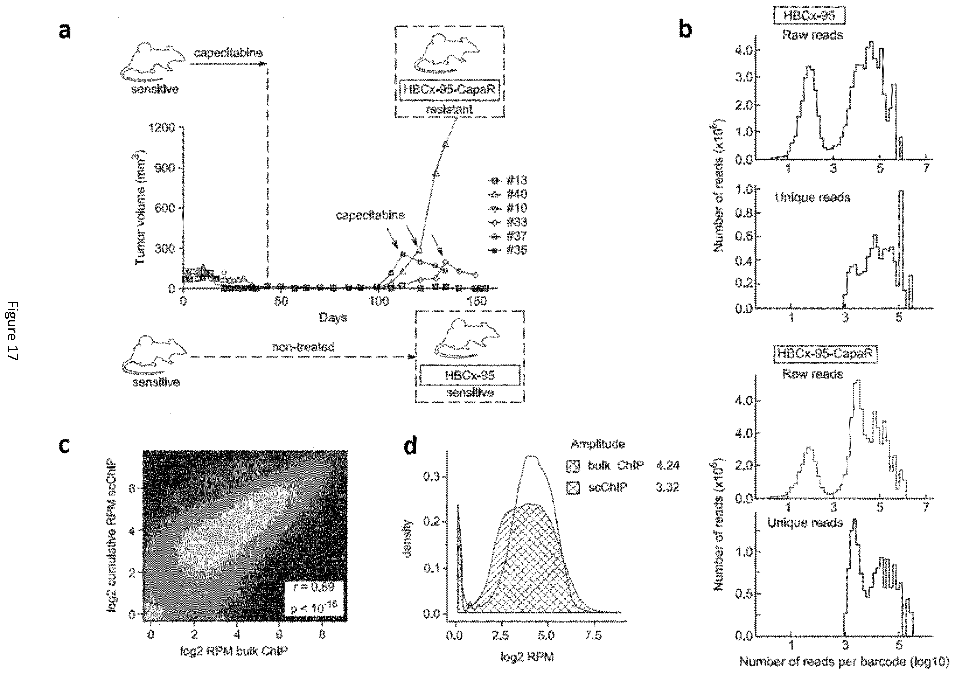

Figure 17 depicts breast tumor model of acquired resistance to Capecitabine. (a) n = 6 mice with HBCx-95 derivatives were treated with Capecitabine for 6 weeks. Graphs represent relative tumor volumes (RTV, mm3) with time. Mice with recurrent tumors were treated for a second round of Capecitabine when PDX reached a volume of over 200 mm3 (mice #35, #40 & #33). Mouse #40 did not respond to Capecitabine and the PDX specimen was extracted at 1080 mm3 and tagged as HBCx-95-CapaR. Samples studied in manuscript are indicated with a dashed rectangle. (b) Histograms of the distribution of scChlP-seq raw and unique sequencing reads per cell in untreated HBCx-95 and Capecitabine-resistant HBCx-95-CapaR PDX. (c) Scatter plot displaying the log2 enrichments in cumulative single-cell versus bulk H3K27me3 ChlP-seq data, calculated based on the counts per million mapped reads in 50 kb genomic bins. Pearson correlation score and p-value are computed genome-wide. (d) Distribution of the log2 enrichments in 50 kb genomic bins for cumulative single-cell and bulk H3K27me3 ChlP-seq data. Amplitude between maximum and minimum log2 enrichments are indicated on the top right for each ChlP-seq method (either bulk or single-cell).

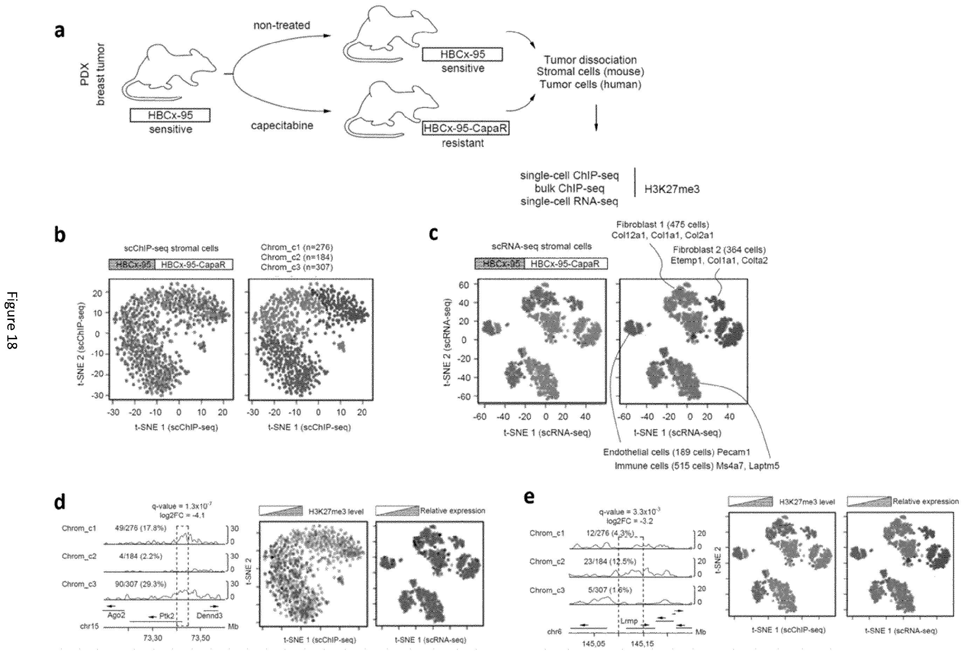

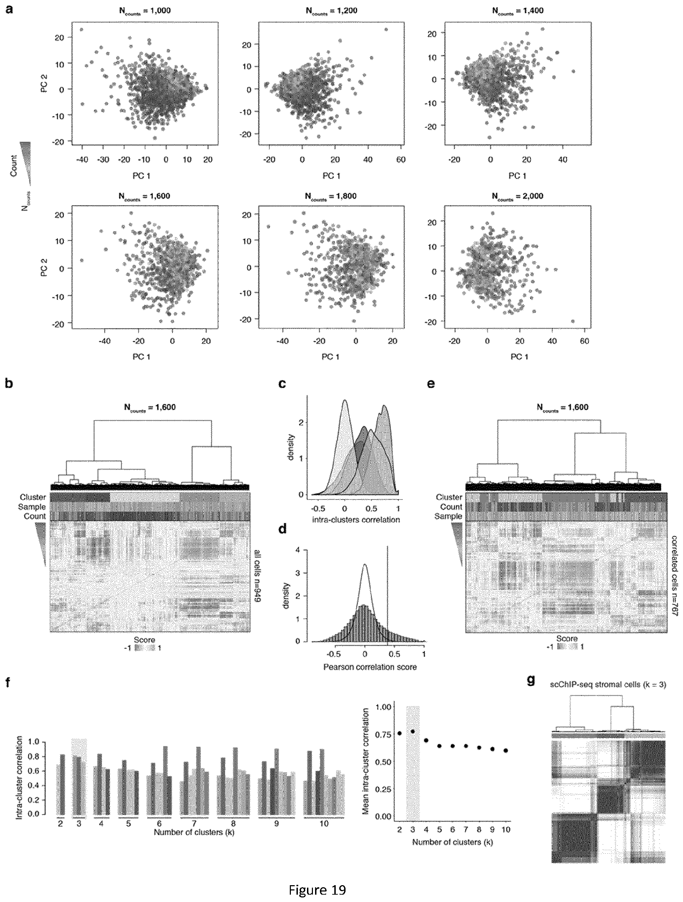

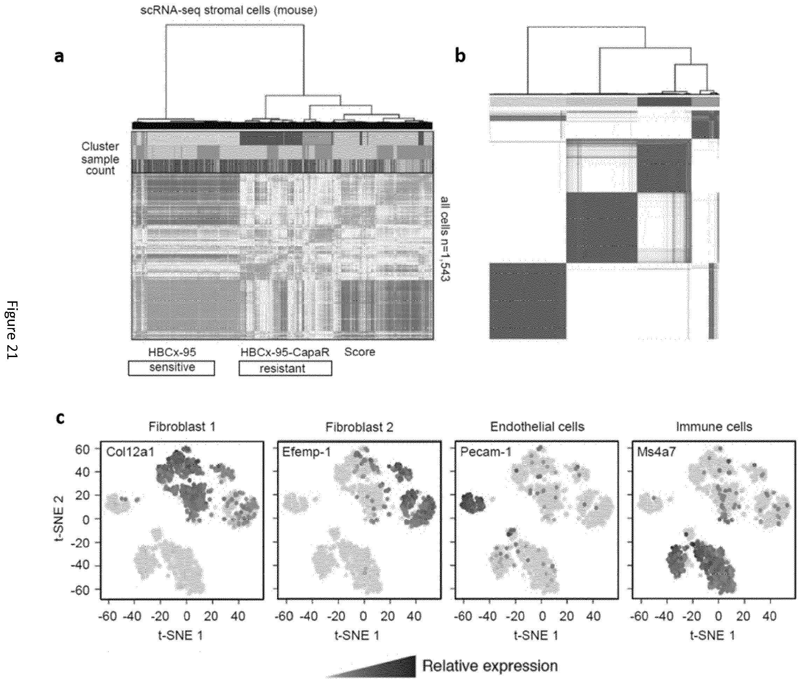

Figure 18 depicts single-cell ChlP-seq profiling of mouse stromal cells reveals cell identities from H3K27me3 chromatin mark. (a) Scheme representing the generation of a PDX model of acquired resistance to chemotherapy (Fig. 17a) and experiments. Sample color code is dark grey for HBCx-95 and light grey for HBCx-95-CapaR. (b) t-SNE representation of scChlP-seq datasets, in different grey shadow according to tumor of origin (sensitive or resistant to Capecitabine, left panel) or consensus clustering results (Fig. 19g). (c) t-SNE representation of scRNA-seq datasets, colored according to tumor of origin or consensus clustering results (Fig. 21b). Marker genes identified by differential expression analysis are indicated for each subpopulation (Fig. 21c). (d-e) Left panels: snapshots of genomic regions, significantly depleted in H3K27me3 in cells from cluster Chrom_c2 (Ptk2) or Chrom_c3 (Lrmp) versus other cells, with cumulative single-cell profiles for each cell type and cluster. The number and the percentage of cells with H3K27me3 enrichment within each cluster are indicated above tracks. Differentially bound regions identified by Wilcoxon signed-rank test are highlighted in grey with the corresponding adjusted p-value and log2 fold-change, the reference cluster is indicated with an asterisk. Middle panels: t-SNE representation of scChlP-seq datasets, points are colored according to H3K27me3 enrichment signal in each cell for Ptk2 and Lrmp loci. Right panels: t-SNE representation of scRNA-seq datasets, points are in different grey shadow according to expression signal in each cell for Ptk2 and Lrmp.

Figure 19 depicts a detailed analysis of scChlP-seq H3K27me3 profiles for stromal cells from the HBCx-95 model. (a) PCA plots with varying minimum number of unique mapped reads per cell showing that H3K27me3 scChlPseq principal components were mainly driven by cell coverage up to 1,600 reads per cell. (b) Hierarchical clustering and corresponding heatmap of cell-to-cell Pearson's correlation scores based on their H3K27me3 profiles for n=949 mouse stromal cells with a minimum coverage of 1,600 unique mapped reads. Cluster membership from hierarchical clustering, sample of origin (green for HBCx-95 and pink for HBCx-95-CapaR) and unique read count are indicated above the heatmap. (c) Distribution of the intra-cluster correlation scores for each cluster identified by hierarchical clustering. (d) Distribution of cell-to-cell Pearson's correlation scores for real scChlP-seq datasets (lighter grey) and randomized datasets (black curve). The upper percentile is indicated by a vertical black line. (e) Hierarchical clustering and corresponding heatmap of cell-to-cell Pearson's correlation scores for 'correlated' cells only, representing 81.8% of all cells. (f) Left: mean of all pairwise correlation scores between cluster's members is plotted for k clusters ranging from 2 to 10. Right: mean intra-cluster correlation score for k clusters ranging from 2 to 10. At k = 3 clusters, the intra-cluster correlation is maximized. (g) Hierarchical clustering and corresponding heatmap of cell-to-cell consensus clustering score for scChlP-seq mouse stromal cells. Consensus score ranges from 0 (white: never clustered together) to 1 (dark blue: always clustered together). Cluster membership is in different grey shadow above the heatmap.

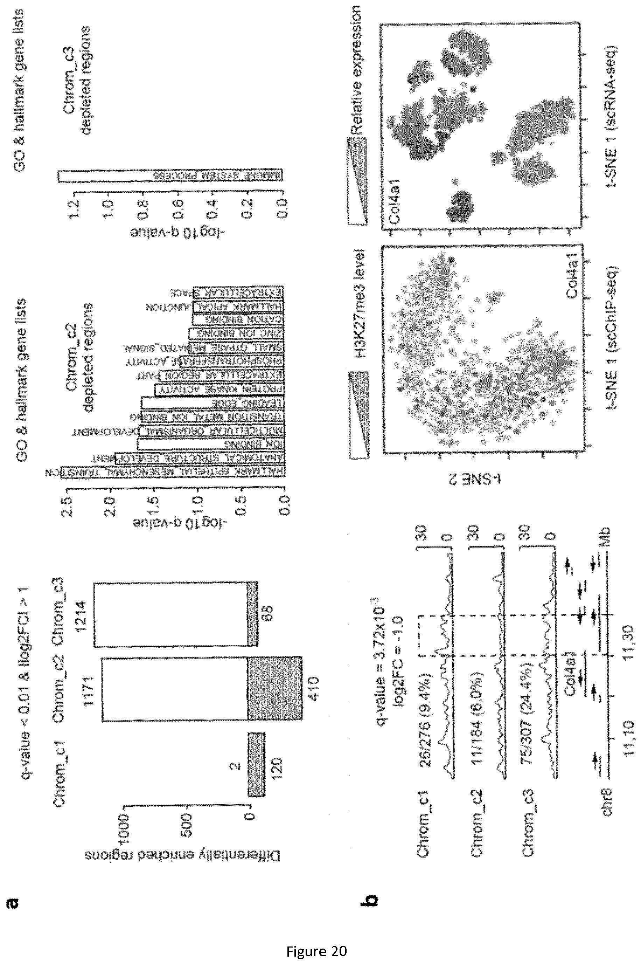

Figure 20 depicts a supervised analysis of single-cell ChlP-seq H3K27me3 profiles of mouse stromal cells from the HBCx-95 model. (a) Left panel: differentially bound regions in cells from each cluster versus all remaining cells identified by Wilcoxon signed-rank test. Genomic regions were considered enriched (dark grey) or depleted (light grey) if the adjusted p-values were lower than 0.01 and the fold change greater than 1. Middle and right panels: barplots displaying the -log10 of adjusted p-values from pathway analysis (using MSigDB, q-value<0.1) for regions depleted in H3K27me3 specifically in Chrom_c2 and Chrom_c3. The top 10 significant gene sets are indicated on the barplots. (b) Left panel: snapshot of a differentially depleted locus for cells from Chrom_c2 (Col4a1) versus all other cells, with cumulative single-cell profiles for each cell type and cluster. The differentially bound region identified by Wilcoxon signed-rank test is indicated in gray with the corresponding adjusted p-value and log2 fold change, calculated in respect to Chrom_c2. The number and the proportion of cells with H3K27me3 enrichment within each cluster are indicated above tracks. Right panels: t-SNE representation of scChlP-seq and scRNA-seq datasets, dots are colored according to H3K27me3 enrichment and expression signals in each cell for Col4a1 locus.

Figure 21 depicts the clustering and supervised analysis of single-cell RNA-seq profiles of mouse stromal cells from the HBCx-95 model. (a) Hierarchical clustering and corresponding heatmap of cell-to-cell Pearson's correlation scores. Cluster membership from consensus clustering, sample of origin (green for HBCx-95 and pink for HBCx-95-CapaR) and unique read count are indicated above the heatmap. (b) Consensus clustering scores for hierarchical clustering of stromal cells. Consensus score ranges from 0 (light grey: never clustered together) to 1 (dark grey: always clustered together). Cluster membership is color coded above the dendrogram. (c) t-SNE plots with dots colored according to the expression signal of each marker gene, representative each cell population, in each cell.

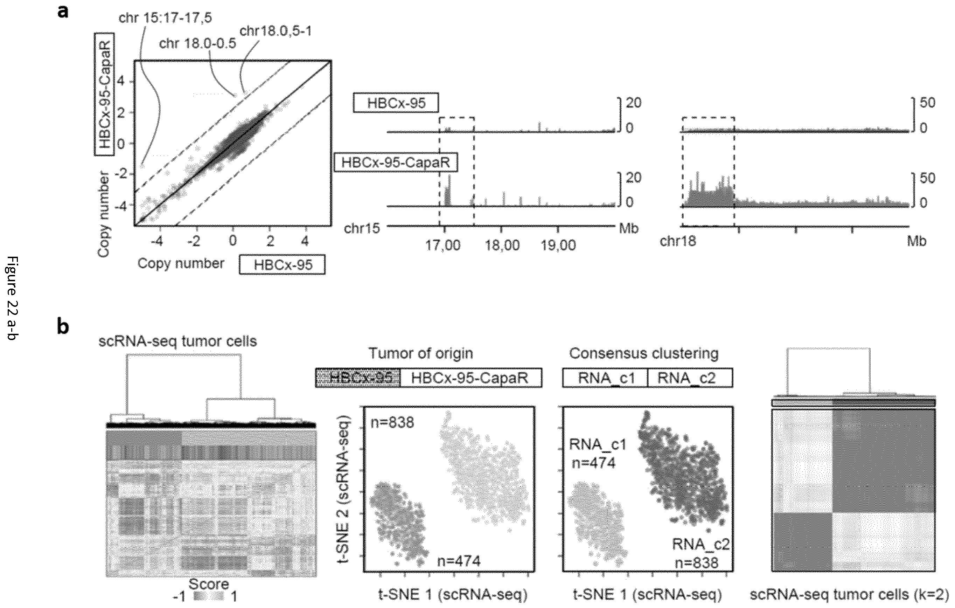

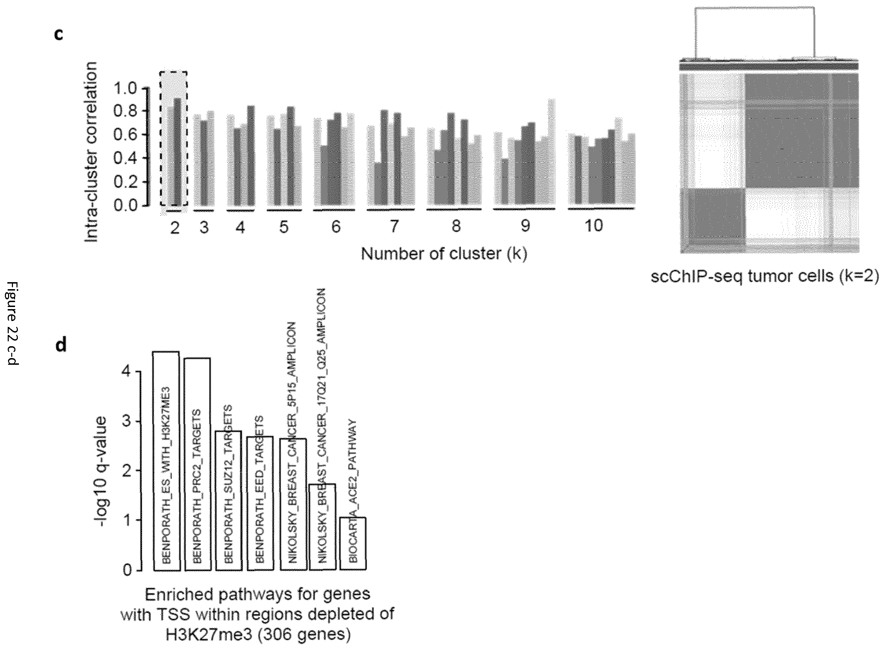

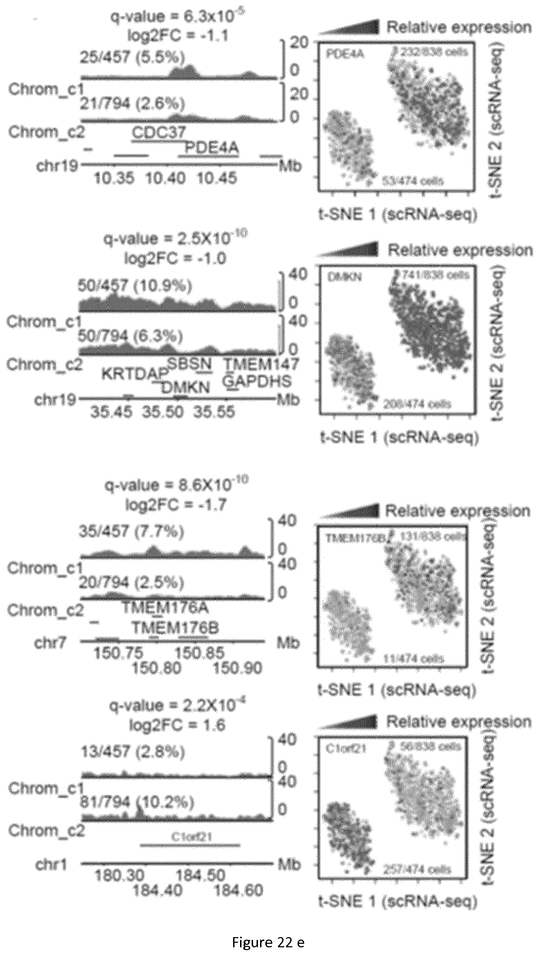

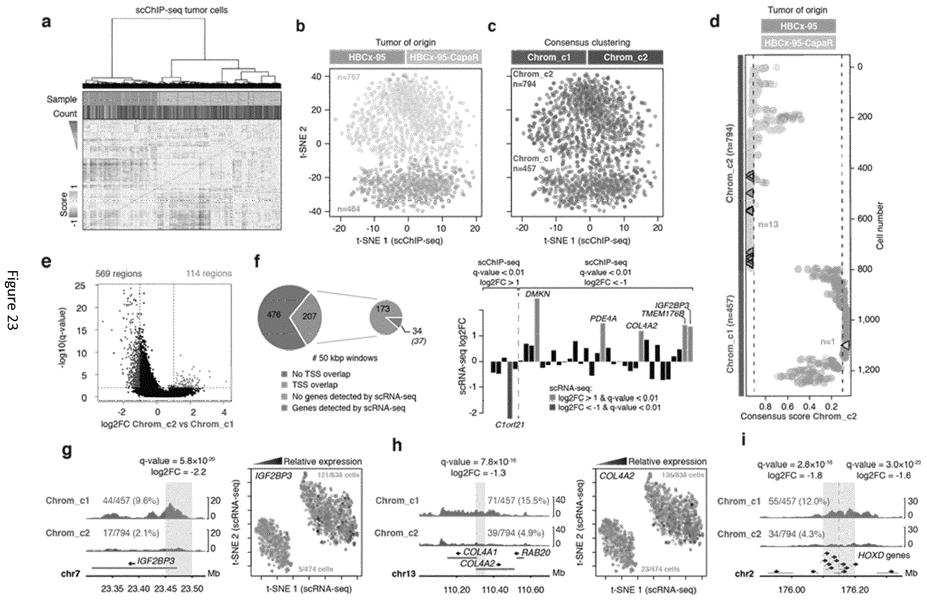

Figure 22 depicts the clustering of single-cell ChlP-seq H3K27me3 profiles and scRNA-seq profiles of human tumor cells from the HBCx-95 model. (a) Left: Plot of copy number in 0.5 Mb non-overlapping regions in Capecitabine-resistant PDX (HBCx-95-CapaR) versus untreated PDX (HBCx-95), obtained from the input of the bulk ChlP-seq experiments. Middle and right: snapshots of loci affected by copy number variation for bulk DNA profiles of Capecitabine-resistance PDX and untreated PDX indicated in grey. (b) Left panel: hierarchical clustering and corresponding heatmap of cell-to-cell Pearson's correlation scores. Sample of origin (dark grey for HBCx-95 and light grey for HBCx-95-CapaR) and unique read count are indicated above the heatmap. Middle panel: t-SNE plots of scRNA-seq tumor cells, dots are in different grey shadow according to the sample of origin and consensus clustering segmentation. Right panel: Consensus clustering scores for hierarchical clustering of scRNA-seq tumor cells. Consensus score ranges from 0 (white: never clustered together) to 1 (dark blue: always clustered together). Cluster membership is in different grey shadows beneath the dendrogram. (c) Consensus clustering analysis for scChlP-seq dataset. Left panel: mean of all pairwise correlation score between cluster's members is plotted for k clusters ranging from 2 to 10. At k = 2 clusters, the intra-cluster correlation is maximized. Right panel: hierarchical clustering and corresponding heatmap of cell-to-cell consensus clustering scores for scChlP-seq on tumor cells (HBCx-95 and HBCx-95-CapaR PDXs). Consensus scores ranges from 0 (light grey: never clustered together) to 1 (dark grey: always clustered together). Cluster membership is in different grey shadow above heatmap. (d) Barplot displaying the -log10 of adjusted p-values from pathway analysis for regions with depletion of H3K27me3 in resistant cells. The gene sets are indicated on the barplot. (e) Left panels: Aggregated H3K27me3 chromatin profiles for Chrom_c1 and Chrom_c2 are shown for the loci identified in Figure 23f, as significantly differentially enriched and expressed. For each window indicated in gray, the log2 fold-change, the adjusted p-value (q-value), the number and the proportion of cells with H3K27me3 enrichment within each cluster are indicated. Right panels: t-SNE representation of scRNA-seq datasets. Dots are in different grey shadow according to expression signal in each cell.

Figure 23 depicts sensitive and drug-resistant specific H3K27me3 chromatin landscapes in PDX model of triple negative breast cancer treated with Capecitabine. (a) Hierarchical clustering and corresponding heatmap of cell-to-cell Pearson correlation scores for scChlP-seq datasets. Sample color code is dark grey for HBCx-95 and light grey for HBCx-95-CapaR and the unique read count is indicated above heatmap. (b-c) t-SNE representation of scChlP-seq datasets, cells are in different grey shadows according to the sample of origin (b) and consensus clustering segmentation (c) (Fig. 22c). (d) Item consensus score in respect to Chrom_c2, a score of 1 corresponds to a cell as highly representative of Chrom_c2 cluster. Dotted lines represent item consensus score of 0.9 relative to Chrom_c2 (left line) or Chrom_c1 (right line). dark grey cells originate from HBCx-95, pink cells from HBCx-95-CapaR. Triangles highlight cells with a consensus score over 0.9 and in opposition to their sample of origin. (e) Volcano plot representing adjusted p-values (Wilcoxon rank test) versus log2 fold-changes for differential analysis comparing chromatin enrichment between Chrom_c2 and c1 (thresholds of 0.01 for q-value and 1 for |log2FC|). (f) Left panel: Pie chart representing the number of differentially enriched windows overlapping a TSS and with detectable transcription. Right panel: Log2 expression fold-change between cells from HBCx-95-CapaR and HBCx-95 for all detected genes (n=37) within differentially enriched loci. Barplot is colored according to log2FC and associated q-value (black for q>0.01, green for significantly under-expressed and red for significantly over-expressed). (g-h) Left panels: aggregated H3K27me3 chromatin profiles for each cluster are shown for IGF2BP3 and COL4A2. The number and the percentage of cells with H3K27me3 enrichment within each cluster are indicated above tracks. Right panels: t-SNE plots representing scRNA-seq datasets, points are in different grey shadows according to cell expression signal for IGF2BP3 or COL4A2. (i) Aggregated H3K27me3 chromatin profiles for HOXD locus depleted in H3K27me3 in Chrom_c2, but with no detectable transcription with scRNA-seq.

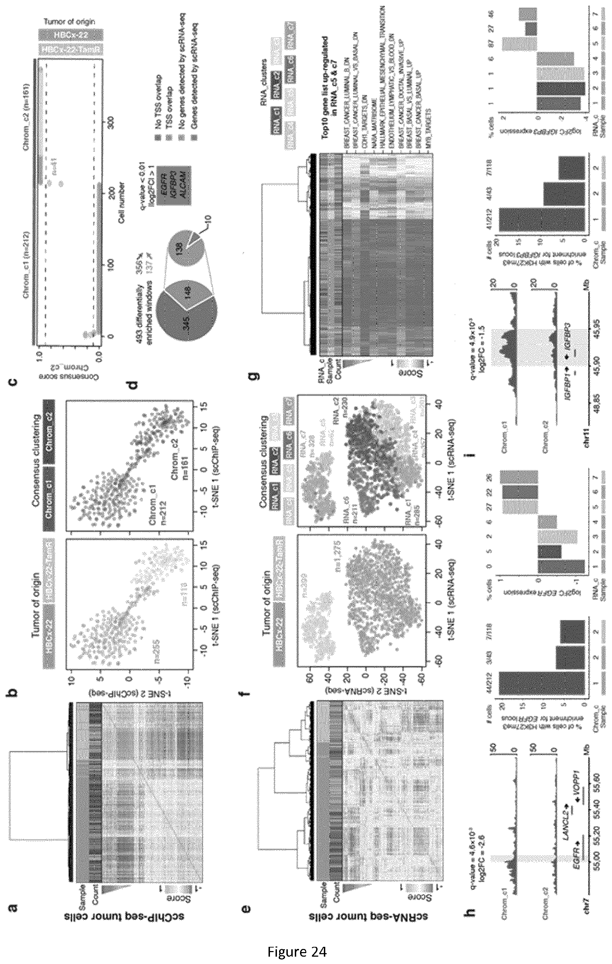

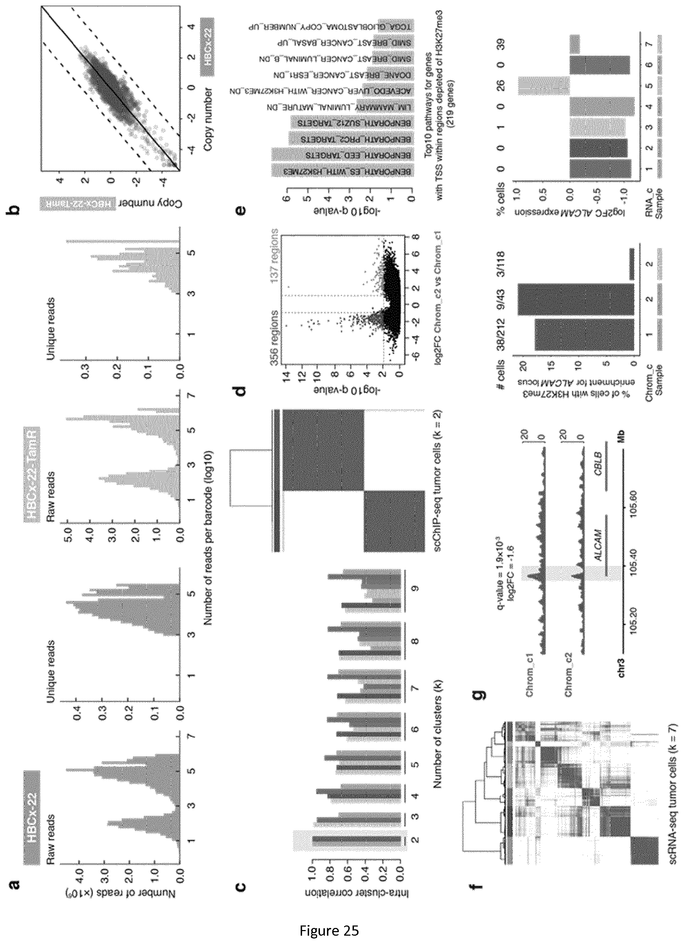

Figure 24 depicts a fraction of cells from sensitive tumor shares H3K27me3 chromatin features with resistant cells in a model of luminal ER+ PDX treated with Tamoxifen. (a) Hierarchical clustering and corresponding heatmap of cell-to-cell Pearson correlation scores for scChlP-seq datasets. Sample of origin is indicated in dark grey for HBCx-22 and light grey for HBCx-22-TamR, the unique read count is indicated above heatmap. (b) t-SNE representation of scChlP-seq datasets, cells colored in different grey shadows according to sample of origin (left) and consensus clustering segmentation (right) (Fig. 25c-d). (c) Item consensus score in respect to Chrom_c2. A score of 1 corresponds to a cell as highly representative of Chrom_c2 cluster. Dotted lines represent item consensus score of 0.9 relative to Chrom_c2 (upper line) or Chrom_c1 (lower line). (d) Pie chart representing the number of significantly differentially enriched (H3K27me3, q<0.01) windows overlapping a TSS and with detectable transcription. (e) Hierarchical clustering and corresponding heatmap of cell-to-cell Pearson correlation scores for scRNA-seq datasets. Sample of origin is indicated in green for HBCx-22 and pink for HBCx-22-TamR, the UMI count is indicated above heatmap. (f) t-SNE representation of scRNA-seq datasets, cells colored in different grey shadows according to sample of origin (left) and consensus clustering segmentation (right) (Fig.25f). (g) Hierarchical clustering of average expression scores per cell for each of the top 10 upregulated pathways (with lowest q-values) in HBCx-22-TamR versus HBCx-22. Sample of origin, RNA cluster and unique read count is indicated above heatmap. (h-i) Left panels: Snapshots for EGFR and IGFBP3 loci of aggregated H3K27me3 chromatin profiles for each cluster. For each window, log2 fold-change and adjusted p-value are indicated. Middle panels: Barplots displaying the percentage of cells with H3K27me3 enrichment in each cluster. The corresponding number of cells is indicated above the barplots. For each cluster, the origin of cells (dark grey for HBCx-22 and light grey for HBCx-22-TamR) is indicated below. Right panels: Barplots displaying the average log2 fold-change in EGFR and IGFBP3 expression level for cells in each cluster versus all remaining cells. The percentage of cells, within each cluster, with detectable EGFR or IGFBP3 expression is indicated above the barplot. For each cluster, origin of cells (dark grey for HBCx-22 and light grey for HBCx-22-TamR) is indicated below.

Figure 25 depicts clustering of single-cell ChlP-seq profiles of human tumor cells from the HBCx-22 model. (a) Histograms of the distribution of scChlP-seq raw and unique sequencing reads per cell in untreated HBCx-22 and Tamoxifen-resistant HBCx-22-TamR PDX. (b) Copy number in 0.5 Mb non-overlapping regions plotted for bulk DNA profiles of Tamoxifen-resistant PDX (HBCx-22-TamR) versus untreated PDX (HBCx-22). No aberrant variation in copy number was identified in this xenograft model. (c) Consensus clustering analysis for scChlP-seq dataset. Left panel: mean of all pairwise correlation score between cluster's members is plotted for k clusters ranging from 2 to 10. At k = 2 clusters, the intra-cluster correlation is maximized. Right panel: hierarchical clustering and corresponding heatmap of cell-to-cell consensus clustering scores for scChlP-seq on tumor cells (HBCx-22 and HBCx-22-TamR PDXs). Consensus scores ranges from 0 (light grey: never clustered together) to 1 (dark grey: always clustered together). Cluster membership is in different grey shadow above heatmap. (d) Volcano plot representing adjusted p-values (Wilcoxon rank test) versus fold-changes for differential analysis comparing chromatin marks between Chrom_c2 and Chrom_c1 (q-value < 0.01 and |log2FC| > 1). (e) Barplot displaying the -log10 of adjusted p-values from pathway analysis for regions with depletion of H3K27me3 in cells from Chrom_c2. The gene sets are indicated on the barplot. (f) Hierarchical clustering and corresponding heatmap of cell-to-cell consensus clustering score for scRNA-seq tumor cells (HBCx-22 and HBCx-22-TamR PDXs). Consensus score ranges from 0 (lightgrey: never clustered together) to 1 (dark blue: always clustered together). Cluster membership is color coded above the heatmap. (g) Left panel: aggregated H3K27me3 chromatin profiles for Chrom_c1 and Chrom_c2 are shown for the ALCAM locus. For each window indicated in grey, the log2 fold-change and the adjusted p-value are indicated. Middle panel: barplot displaying the proportion of cells with H3K27me3 enrichment in each cluster. The corresponding number of cells is indicated above the barplot. For each cluster, the origin of cells (dark grey for HBCx-22 and light grey for HBCx-22-TamR) is indicated below. Right panel: barplot displaying the average log2 fold-change for ALCAM expression level for cells in each cluster versus all remaining cells. The percentage of cells, within each cluster, with detectable ALCAM expression is indicated above the barplot. For each cluster, the origin of cells (dark grey for HBCx-22 and light grey for HBCx-22-TamR) is indicated below.

### DETAILED DESCRIPTION SECTION

The inventors have developed an improved single-cell ChIP method based on droplet microfluidics, which leads to a 5- to 10-fold increase in the number of enriched loci per individual cell compared to Drop-ChIP technology disclosed in Rotem (see Figure 7). The method allows with high sensitivity and precision to assess at the single cell level, histone modifications, DNA modified bases (including modified nucleotides for identifying ongoing DNA replication events at the single cell or any biological element level), chromatin/DNA associated factors. This method is applicable for the identifications of cell populations or any biological element having distinct features from another one, these features being histone and/or DNA modification, presence of factors. The presence or absence of these elements are then potentially indicative of variation in gene expression, and thus can be used as biomarkers, therapeutics targets to revert the changes.

It is well understood that cells can mean the nucleic, as compartment of chromatin structure. The cell or nuclei or any biological element can be fixed biological elements. Examples of fixative agents include aldehyde (including but not limited to formaldehyde, paraformaldehyde), alcohol (including but not limited to ethanol and methanol), oxidizing agents, mercurial, picrates, Hepes-glutamic acid buffer-mediated organic solvent protection effect (HOPE) fixatives.

As in Drop-ChIP, droplets containing cells and droplets containing barcodes are separately produced before being re-injected and merged one-to-one in a dedicated microfluidic fusion device (see Figure 1). However, the present method differs over Rotem in at least two aspects characterizing the barcode strategy. Firstly, the inventors replaced soluble barcodes emulsified from microtiter plates containing oligonucleotides by hydrogel beads (or any solid support) carrying millions of a unique or most abundant DNA sequence. Secondly, the novel design of the barcode structure according to one aspect of the invention allows linear amplification of all barcoded nucleosomes and not only symmetrically barcoded nucleosomes on both ends as in Rotem. Thirdly, the barcode design includes additional features increasing the efficiency of the barcoding of the nucleic acids of interest. These features include the addition of protective moiety to the 'incomplete barcode' preventing their attachment to the nucleic acid of interest. In another aspect, the barcode can include protectives bases on the full length oligos (protectives bases include, but are not limited to phosphorotioate, LNA/BNA, nucleotides phosphoramitidites, synthetic cycles, non 3'OH or 5'P bases, 2'-O-methyl-DNA/RNA), these protectives bases will protect the full length barcode while the non-full length barcode can be digested with exonuclease.

An additional set of barcodes, called 'experimental barcodes' can be added in order to multiplex different experiments in a single immuno-precipitation reaction. Subsequent bioinformatics analysis will allow to demultiplex the experimental conditions based on the sequence of the 'experimental barcode'.

It is well understood that the barcode is a nucleic acid sequence that can distinguish features specifics from nucleic acid originating from one compartment to another. Production of these barcodes are known by people skilled in the art and can represent a random sequence (flanked or not with known sequences), or generated by split pool synthesis (Klein et al., Cell, 2015).

Additionally, the method according to the invention is characterized by a synchronization/pausing step, which limits cell-to-cell variation in chromatin digestion between droplets.

The aforementioned advantages are disclosed hereinafter in aspects and embodiments characterizing the invention. Implementation of the invention is provided in examples and figures.

A first aspect of the invention provides a method of identifying one or more genomic region(s) of interest using a microfluidic system, the method comprising the steps of:
a. providing at least a droplet of first type, wherein said droplet of first type comprises
   i. a biological element,
   ii. a lysis buffer, and
   iii. a nuclease,
b. collecting said droplet of first type under conditions that temporarily inactivate said nuclease,
c. incubating said droplet of first type, thereby reactivating said nuclease,
d. providing at least a droplet of second type, wherein said droplet of second type comprises a nucleic acid sequence,
e. merging said droplets of first and second type, thereby generating a droplet of third type,
f. incubating said droplet of third type, thereby linking said nucleic acid sequence to at least one genomic region(s) of interest,
g. sequencing said one or more genomic region(s) of interest.

The method of the invention is carried out in a microfluidic system. In the context of the invention, the term "microfluidic system" refers to a system or device having one or more channels and/or chambers that are generally fabricated on the micron or submicron scale.

The method of the invention is characterized by the presence of droplets of first, second and third types. As used herein, the terms "first", "second" and "third" associated to droplets are used to discriminate droplets according to their content. As the method is performed in a microfluidic system, the term "droplet" also refers to "microfluidic droplet". Therefore, in the context of a microfluidic system, the term "droplet" also refers to an isolated portion of a first fluid that is surrounded by a second fluid, where the first and second fluids are immiscible.

According to the stages or step of the method of the invention, the droplets may be contained in a microfluidic system (on-chip) or in a collector device (off-chip) separate from the microfluidic system. The droplets may have a spherical or non-spherical form.

In one embodiment of the invention, the droplet has a volume ranging from about 20pl to about 100pl. Preferably, the droplet has a volume ranging from about 30pl to about 70pl. More preferably, the droplet has a volume ranging from about 40pl and about 50pl. Ideally, the droplet has a volume of about 45pl. As used herein, the term "about" refers to a range of values ±10% of a specified value.

As used herein, the term "lysis buffer" refers to buffer that is capable of lysing biological cells. The meaning of the term "lysis buffer" is within the common general knowledge of a person skilled in the art.

As used herein, the term "genomic region" refers to DNA or RNA encoded nucleic acid sequences.

As used herein, the term "nuclease" refers to an enzyme agent capable of cleaving a phosphodiester bond connecting nucleotide residues in a nucleic acid molecule. The nuclease may digest double-stranded, single-stranded, circular and linear nucleic acid molecules. In the context of the invention, a nuclease may be an endonuclease, which cleaves a phosphodiester bonds within a polynucleotide chain, or an exonuclease, which cleaves a phosphodiester bond at the end of the polynucleotide chain, may be a transposase. A nuclease may also be a site-specific nuclease, which cleaves a specific phosphodiester bond within a specific nucleotide sequence, e.g. a recognition sequence. A non-limiting example of nuclease is Micrococcal nuclease (MNase). In a particular embodiment, the nuclease is a Micrococcal nuclease (MNase).

As used herein, the term "biological element" can refer to a single cell, a nucleus, a nucleic acid containing organelle (e.g. mitochondria) and can be obtained from an organism, a human or a non-human subject. In the latter case, the non-human subject is not limited to a mammal subject.

Performing enzymatic assays on individual biological element in droplets is challenging as cells are processed sequentially with different time scales. For example, the encapsulation step of the cells or any biological element lasts about 20min, which is in the same order of magnitude as the incubation step. Therefore, cells or any biological element encapsulated in droplets at the beginning will be longer in contact with nuclease than the cells or any biological element encapsulated in droplets at the end of the production. Similar observation can be made regarding the re-injection of the droplets in the fusion device (see general scheme in Figure 1). Indeed, the fusion of the two emulsions can last between 1h to 4h depending on the design of the experiment, meaning that some droplets containing fragmented DNA "wait" for hours before being fused and their MNase inactivated by EGTA. Consequently, synchronizing and pausing enzymatic activity are critical in order to avoid the introduction of chromatin digestion variation between individual cells or any biological element.

Notably, in conventional bulk ChlP-seq assays, the inactivation of a nuclease occurs immediately after nuclease incubation with addition of EGTA. Differently, in single-cell ChlP-seq assays, EGTA cannot be added immediately within the droplets and the nuclease is only inactivated after fusion with the barcodes-containing droplets.

In order to control and limit cell-to-cell or any biological element variation in chromatin digestion, the inventors introduced a step of collecting droplets of first type under conditions that temporarily inactivate said nuclease. Said collection step aimed to synchronize/pause the nuclease activity in the droplets is performed before each incubation step. The inventors identified that the droplet compartment render the MNase enzyme sensitive to temperatures changes and is capable of selectively blocking/reactivating and re blocking the enzyme activity. Such tight control of nuclease activity is not possible in bulk. This impact is suspected not being dependent on the sole MNase activity but to any enzyme.

Therefore, according to another embodiment, the method further comprises a step of collecting droplets of first type under conditions that temporarily inactivate said nuclease before step (e).

In yet another embodiment, the conditions of step (b) comprise selecting a temperature degree ranging from -20°C to 10°C and condition of step (c) comprise selecting a temperature degree ranging from 20°C to 40°C.

Droplets can be incubated outside the microfluidic system (off-chip) for single-cell chromatin fragmentation. As the lysis occurs in droplets, the nuclear DNA from lysed cells is accessible for nuclease enzyme. Therefore, the kinetic of the digestion is particularly important to yield preferentially mono-nucleosomes, which are retained in the droplet.

In a particular embodiment, the incubation step (c) is timed to obtain nuclear DNA fragmented into mono-nucleosomes.

In yet another embodiment, the one or more genomic region(s) of interest is a modified genomic region.

According to the invention, the modified genomic region comprises a protein complex associated with a nucleic acid sequence and/or a nucleic acid sequence. In a particular embodiment, the modified genomic region is a modified mono-nucleosome. In another embodiment, the modified genomic region is a transcription factor binding site, a chromatin modifier binding site, a chromatin remodeler site, a histone chaperone binding site.

According to the invention, the modified genomic region can also comprise a post-translational modification selected from the group comprising acetylation, amidation, deamidation, carboxylation, disulfide bond, formylation, glycosylation, hydroxylation, methylation, myristoylation, nitrosylation, assuccinylation, butyrylation, phosphorylation, prenylation, ribosylation, sulphation, sumoylation, ubiquitination and derivatives thereof.

According to the invention, the modified genomic region can also comprise histone variants selected from the group comprising CENP-A/CID/cse4 (epigenetic marker of the centromere), H3.3 (transcription), H2A.Z/H2AV (Transcription/double strand break repair), H2A.X (Double strand break repair/meiotic remodeling of sex chromosomes), macroH2A (Gene silencing/X chromosome inactivation), H2A.Bbd (Epigenetic mark of active chromatin), H3.Z (Regulation of cellular response to outside stimuli), H3.Y (Regulation of cellular response to outside stimuli).

According to the invention, the modified genomic region comprises a modified DNA sequence selected from the group comprising methylation and its derivatives, modified nucleotides like EdU, BrdU, IdU, CldU and others. The most common way of modifying bases is the addition of a methyl mark, and across species, methylation has been found on cytosines and adenines, resulting in 5mC, N4-methylcytosine (N4mC), or 6-methyladenine (6mA), 5-hydroxymethylcytosine (5hmC), 5-formylcytosine (5fC) and 5-carboxylcytosine (5caC).

As introduced above, a remarkable limitation of the method disclosed in Rotem is that only symmetrically indexed nucleosomes can be amplified and can be part of the sequencing library. This requirement dramatically increases the stringency of the system and imposes a strong selection on the nucleosomes, which are limited to those with both ends ligated to a barcode. In contrast with the Drop-ChIP method, the inventors have surprisingly found that indexing nucleosomes from only one end would increase the single-cell coverage and ultimately the capacity of the system to distinguish more subtle variations between single-cell chromatin profiles.

In yet another embodiment, the nucleic acid sequence is asymmetrically linked to said at least one or more genomic region(s) of interest.

As used herein, the term "asymmetrically linked" refers to the presence of at least one barcode linked to a genomic region of interest, whereby the linkage is to only one of the two extremities of the genomic region of interest.

In a second aspect of the invention, there is provided a nucleic acid sequence comprising:
a. at least one index sequence,
b. a sequencing adaptor, and
c. at least one protecting function positioned at 3'- and/or 5'-end.

As used herein, the term "nucleic acid sequence" refers to a single- or double-stranded nucleic acid. In yet another embodiment, a "nucleic acid sequence" can be DNA or RNA. In a preferred embodiment, the "nucleic acid sequence" is a double-stranded DNA. In some embodiments, the "nucleic acid sequence" comprises a double-stranded DNA comprising a first strand barcode and a second strand barcode. In some embodiments, the first strand barcode and second strand barcode comprise complementary sequences. In some embodiments, the first strand barcode and second strand barcode comprise non-complementary sequences.

As used herein, the term "index sequence" refers to a unique nucleotide sequence that is distinguishable from any other index sequences as well as from any other nucleotide sequences within the nucleic acid sequence, wherein it is comprised. An "index sequence" can be a random or a specifically designed nucleotide sequence. An "index sequence" can be of any sequence length. A nucleic acid sequence according to the second aspect of the invention, can be attached to a genomic region of interest (target) to tag a species that requires to be identified and/or to discriminate different members of the tagged species within a population thereof. Accordingly, in the context of the invention, the terms "index sequence" and "barcode" can be used interchangeably.

As used herein, the term "sequencing adapter" refers to an oligonucleotide of known sequence, the ligation or incorporation of which to a polynucleotide or a polynucleotide strand of interest enables the generation of products of said polynucleotide or said polynucleotide strand of interest ready for amplification.

In one embodiment of the second aspect of the invention, the nucleic acid sequence further comprises at least one cleavage site.

As used herein, the term "cleavage site" refers to a target region of the nucleic acid sequence that is susceptible of being cleaved by any means, including but not limited to enzymes, that is capable of cleaving a single or double-stranded nucleic acid sequence. In the context of the invention, the "cleavage site" can serve to cleave or otherwise release a portion of the nucleic acid sequence. The "cleavage site" is recognized by a cleaving agent, which can be natural, synthetic, unmodified or modified.

In one embodiment of the invention, the protecting function is selected from the group comprising a spacing element on a 3'-end and a dideoxy-modified base on 5'-end. In the context of the invention, a suitable non-limiting spacing element is a three carbon spacer (C3 spacer).

In yet another embodiment of the invention, the at least one cleavage site is a restriction site comprising a palindromic region.

As used herein, the term "restriction site" refers to a site that is recognized by a restriction enzyme, e.g. an endonuclease. A person skilled in the art is familiar with restriction endonucleases and their restriction sites. Non-limiting examples of restriction site include BamHI, Bsrl, Notl, Xmal, PspAl, Dpnl, Mbol, Mnll, Eco57I, Ksp632I, Dralll, Ahall, Smal, Mlul, Hpal, Apal, Bcll, BstEII, Taql, EcoRI, Sacl, Hindll, Haell, Drall, Tsp509I, Sau3AI, PacI.

In yet another embodiment of the invention, the nucleic acid sequence is suitable for use in a method according to the first aspect of the invention and its embodiments.

In another embodiment of the invention, the nucleic acid sequence is suitable for use in profiling the epigenetic state in a sample obtained from a subject.

As used herein, the term "sample" refers to a biological sample.

As used herein, the term "subject" refers to a human or a non-human subject. In the latter case, the non-human subject is not limited to a mammal subject.

The method according to the invention can find different application in the identification of genes, and factors involved in the diagnosis and/or prognosis of a diseased state in a subject, as well as methods for the diagnosis and/or prognosis of a diseased state in a subject and for controlling the effect of therapeutics molecules on chromatin.

In the context of the invention, a diseased state can refer to any modification involving nucleosomes or nucleic acid sequences, as well as positioning of proteins impacting chromatin structure, regulation and function. As used herein, the expression "diseased state" also encompasses an abnormal rate of cell proliferation so that the treatment of the disease requires modulation of the cell cycle. Examples of proliferative diseases include, without being limited thereto, cancer.

The method according to the invention can be used for the *in vitro* diagnosis and/or prognosis of drug resistance, wherein single cell chromatin states are profiled in cells obtained from a subject by using a microfluidic system, the method comprising the steps of:
b. providing at least a droplet of first type, wherein said droplet of first type comprises
   a. a biological element,
   b. a lysis buffer, and
   c. a nuclease,
c. collecting said droplet of first type under conditions that temporarily inactivate said nuclease,
d. incubating said droplet of first type, thereby reactivating said nuclease,
e. providing at least a droplet of second type, wherein said droplet of second type comprises a nucleic acid sequence,
f. merging said droplets of first and second type, thereby generating a droplet of third type,
g. incubating said droplet of third type, thereby linking said nucleic acid sequence to one or more genomic region(s) of interest,
h. sequencing said one or more genomic region(s) of interest.

The drug resistance might be an emerging drug resistance and/or a present drug resistance.

The emergence of the drug resistance can be due to epigenetic heterogeneity.

Single-cell chromatin profiling appears as a unique tool to probe the role of heterogeneity and dynamics of chromatin states within any complex biological system: in addition to cancer it can be applied to other diseases, notably auto-immune disease, infectious, metabolic diseases and healthy systems, notably to study cellular differentiation and development, and immune surveillance.

The method according to the invention can be used to determine the patient stratification, wherein the drug-resistance is associated with distinct chromatin states using single-cell epigenetic profiling in microfluidic droplets is required.

In embodiments of the invention, it is provided a method for the diagnosis and/or prognosis of drug resistance in a subject in a diseased state and/or suspected to be in a diseased state.

In embodiments of the invention, it is provided a method for the diagnosis and/or prognosis of drug resistance in a healthy subject.

According to the invention the diagnosis and/or prognosis of drug resistance in a subject, can be performed at a timepoint before, during, or after said subject underwent a treatment or therapy. The diagnosis and/or prognosis can also be performed at any other timepoint. The treatment or therapy might be a treatment or therapy with a chemotherapeutic drug, a chemical drug or biologics drugs like an antibody (and derivatives or fragments thereof) including anti-immune checkpoint treatment, like a chemokine, like an hormone, like a cytokine (and derivatives thereof) or like cell therapy consisting of TILs (tumor infiltrated T cells) injection, CAR T cells (chimeric associated Antigen), CAR NK cells, TCR therapy (in the form of soluble or cellular therapy), like vaccination (cancer vaccine, virus vaccines, Dendritic Cells Therapy inducing vaccination), like oncolytic viruses, like nanoparticles.

In embodiments of the invention, it is provided a method for the diagnosis and/or prognosis of a subject presenting a drug-resistance and/or suspected of having a drug resistance.

The subject may be a subject in a diseased state and/or suspected of having a diseased state or a healthy subject.

As used herein, the term "diagnosis" refers to the determination as to whether a subject is likely to present or develop a drug resistance. The term "diagnosis" as used herein refers to methods by which the person skilled in the art can estimate and/or determine the probability ("a likelihood") of whether or not a subject is suffering from a drug resistance, such as resistance to a therapeutic agent, a chemotherapeutic drug, a chemical drug or biologics drugs like an antibody (and derivatives or fragments thereof) including anti-immune checkpoint treatment, like a chemokine, like an hormone, like a cytokine (and derivatives thereof) or like cell therapy consisting of TILs (tumor infiltrated T cells) injection, CAR T cells (chimeric associated Antigen), CAR NK cells, TCR therapy (in the form of soluble or cellular therapy), like vaccination (cancer vaccine, virus vaccines, Dendritic Cells Therapy inducing vaccination), like oncolytic viruses, like nanoparticles. In the case of the invention, "diagnosis" includes using the results of an assay, most preferably a scChlP.

As used herein, the term "prognosis" refers to the prediction of the likelihood of the drug resistance attributable death or progression of a disease such as cancer, including recurrence and metastatic spread, inflammation, infectious disease, auto immune disease, metabolic disease, genetic e non-genetic disease.

The method according to the invention may use single cells deriving from a body sample.

In embodiments of the invention the body sample is fluid and/or solid. As used herein, a body sample can be from tissue, blood, serum, plasma, spittle, stool, urine, breast, lung, colon, gut, brain, colon, kidney or any other body sample.

According to the invention, the one or more genomic region of interest is a modified genomic region. The modified genomic regions comprise a nucleic acid sequence and/or a protein complex associated with a nucleic acid sequence. The modified genomic region comprises a post-translational modification selected from the group comprising acetylation, amidation, deamidation, carboxylation, disulfide bond, formylation, glycosylation, hydroxylation, methylation, myristoylation, nitrosylation, assuccinylation, butyrylation, phosphorylation, prenylation, ribosylation, sulphation, sumoylation, ubiquitination and derivatives thereof.

In a preferred embodiment, the cells are treated with chemotherapeutic drug, a chemical drug or biologics drugs like an antibody (and derivatives or fragments thereof) including anti-immune checkpoint treatment, like a chemokine, like an hormone, like a cytokine (and derivatives thereof) or like cell therapy consisting of TILs (tumor infiltrated T cells) injection, CAR T cells (chimeric associated Antigen), CAR NK cells, TCR therapy (in the form of soluble or cellular therapy), like vaccination (cancer vaccine, virus vaccines, Dendritic Cells Therapy inducing vaccination), like oncolytic viruses, like nanoparticles. In a more preferred embodiment, the cells are treated with a chemotherapeutic drug.

In one embodiment of the invention, the cells are treated with Tamoxifen, which targets the estrogen receptor, and Capecitabine, a classical chemotherapy inhibiting the synthesis of thymidine monophosphate.

In one embodiment of the invention the, the cells are untreated.

As used herein, a "diseased state", refers to a disease, such as cancer, or infectious disease, auto-immune disease, metabolic disease, inflammation disease, genetic and non-genetic diseases.

In one embodiment of the invention, the diseased state of the subject comprises cancer, infectious disease, auto-immune disease, inflammation disease, metabolic disease genetic disease, non-genetic diseases.

In one embodiment of the invention, the diseased state comprises cancer at any stage, from non-detectable to stage IV. In one embodiment of the invention, the cancer comprises any type of cancer, such as solid and/or liquid cancer.

In a preferred embodiment of the invention the diseased state of the subject is breast cancer.

According to the invention the subject might be a male or a female subject; in a preferred embodiment of the invention the subject is a female subject.

In a further embodiment according to the invention, the single cell chromatin state has lost chromatin marks for genes that promote drug resistance. The chromatin marks comprise the distinct histone modifications H3K4me3, H3K27ac and H3K27me3. The H3K4me3 mark is expected to allow a gene to not be permanently silenced and activated when needed. The H3K27me3 mark is expected to silence the gene. The H3K27ac mark at gene enhancer is expected to promote gene activation.

In one embodiment the single cell chromatin state has lost chromatin marks for genes that likely leads to drug resistance. Chromatin marks comprise histone modifications H3K4me3 and H3K27me3.

As used herein "chromatin marks", "marks for genes" or "mark" refer to DNA and histone modifications, which contribute to the definition of epigenomic signatures within distinct chromatin states, which are highly indicative of cell type and tissue identity. The genome-wide profiling of these marks can be leveraged to understand the global landscape of genome regulation and then, for example, distinguish epigenomic differences in the context of normal and disease cell states. The skilled person knows several chromatin marks as for example disclosed in (Consortium Epigenomics 2015, Nature 518(7539):317-329) or other studies.

In one embodiment the single cell chromatin state has gained a mark, wherein said mark has a desilencing impact.

As used herein, the terms "cancer" and "tumor" are used interchangeably and relate to malignant neoplasia. Examples of malignant neoplasia include solid and hematological tumors. Solid tumors are exemplified by tumors of the breast, bladder, bone, brain, central and peripheral nervous system, colon, endocrine glands (e.g. thyroid and adrenal cortex), esophagus, endometrium, germ cells, head and neck, kidney, liver, lung, larynx and hypopharynx, mesothelioma, ovary, pancreas, prostate, rectum, renal, small intestine, soft tissue, testis, stomach, skin, ureter, vagina and vulva. Malignant neoplasia includes inherited cancers exemplified by Retinoblastoma and Wilms tumor. In addition, malignant neoplasia includes primary tumors in said organs and corresponding secondary tumors in distant organs ("tumor metastases"). Hematological tumors are exemplified by aggressive and indolent forms of leukemia and lymphoma, namely non-Hodgkins disease, chronic and acute myeloid leukemia (CML / AML), acute lymphoblastic leukemia (ALL), Hodgkins disease, multiple myeloma and T-cell lymphoma. Also included are myelodysplastic syndrome, plasma cell neoplasia, paraneoplastic syndromes, cancers of unknown primary site as well as AIDS related malignancies.

Cancer is typically labelled in stages from I to IV, in order to determine the extent to which a cancer has developed, in terms of growing and spreading at the time of the diagnosis. In stage I cancers are localized to one part of the body and can be removed by surgery. In stages II and III cancers are locally advanced and can be treated by chemotherapy, radiation or surgery. In stage IV cancers have metastasized or spread to other organs and can be treated by chemotherapy, radiation or surgery. (Stage V is only used in patients affected by Wilm's tumor, wherein both kidneys are affected.)

As used herein, "metabolic disease" includes, but not limited to, Metabolic syndrome X, Inborn error of metabolism, mitochondrial diseases, phosphorus metabolism disorders, porphyria, proteostasi's deficiencies, metabolic skin diseases, wasting syndrome, water-electrolyte imbalance, metabolic brain diseases, disorders of calcium metabolism, DNA repair-deficiency disorders, iron metabolism disorders, lipid metabolism disorders, malabsorption syndromes.

As used herein, "autoimmune disease" includes, but not limited to, multiple sclerosis, amyloidosis, ankylosing spondylitis, anti-GBM/Anti-TBM nephritis, antiphospholipid syndrome, autoimmune angioedema, Autoimmune dysautonomia, autoimmune encephalomyelitis, autoimmune hepatitis, Polyarteritis nodosa, Polyglandular syndromes type I, II, III, Polymyalgia rheumatica, Polymyositis Myositis, Narcolepsy, Pyoderma gangrenosum, Raynaud's phenomenon, Interstitial cystitis (IC), Juvenile arthritis, Juvenile diabetes (Type 1 diabetes), Juvenile myositis (JM)Reactive Arthritis, neonatal Lupus, Neuromyelitis optica, Celiac disease, Chagas disease, Primary biliary cirrhosis, Primary sclerosing cholangitis, Progesterone dermatitis, Psoriasis, Psoriatic arthritis Chronic inflammatory demyelinating polyneuropathy (CIDP), Transverse myelitis, Type 1 diabetes, Ulcerative colitis (UC)Chronic recurrent multifocal osteomyelitis (CRMO), Churg-Strauss Syndrome (CSS) or Eosinophilic Granulomatosis (EGPA), Neutropenia, Ocular cicatricial pemphigoid, Optic neuritis, Palindromic rheumatism.

As used herein, "genetic disease" includes, but not limited to, to Severe Combined Immunodeficiency (SCID), Sickle cell disease, Skin Cancer, Wilson Disease, Turner syndrome, Spinal Muscular Atrophy, Tay-Sachs, Thalassemia, Trimethylaminuria, myotonic Dystrophy, Neurofibromatosis, Noonan Syndrome, Dercum Disease, Down Syndrome, Duane Syndrome, Duchenne Muscular Dystrophy, Factor V Leiden Thrombophilia, Autism, Autosomal Dominant Polycystic Kidney Disease, Breast cancer, Charcot-Marie-Tooth.

As used herein, "inflammation disease" includes, but not limited to, allergy, asthma, autoimmune diseases, coeliac disease, glomerulonephritis, hepatitis, inflammatory bowel disease.

### EXAMPLES

### Microfluidic workflow of the single-cell ChIP-seq procedure

A general scheme of the microfluidic method according to the invention is depicted in Figure 1. (a) Cells are compartmentalized in 45pl droplets with the reagents necessary for cell lysis and chromatin fragmentation. In parallel, hydrogel beads carrying DNA barcodes are encapsulated in 100pl droplets with ligation reagents and EGTA that inactivates MNase. The two emulsions are reinjected in a fusion device, the barcode-containing droplets (100pl) and the nucleosome-containing droplets (45pl) are paired asymmetrically and merged by electro-coalescence triggered by an electric field. Fused droplets are scanned one-by-one by a laser beam to analyze the composition of each droplet in real time. (b) Emulsion of fused droplets is collected and incubated off-chip for nucleosomes barcoding in droplets. Barcodes are released from the beads by photo-cleavage and ligated to the nucleosomes. The content of droplets is combined, immunoprecipitated and the enriched DNA is sequenced. The deconvolution of the barcode-associated reads attributes all sequences to their originating cell in order to reconstruct single-cell chromatin profile.

### Synchronizing and pausing chromatin fragmentation in droplets

Cells are compartmentalized in 45pl droplets with a digestion mix comprising the lysis buffer and the MNase (see Figure 1). After complete lysis, chromatin is released into the droplets and accessible to get cleaved by the MNase. This section presents a typical calibration of MNase activity in droplets in order to yield preferentially fragments of the size of a nucleosome. However, performing enzymatic assays in droplets might be challenging as cells are processed individually with different time scales. Consequently, fine-tuning enzymatic activity is necessary to avoid chromatin digestion discrepancy between droplets and single-cells.

### Compartmentalization of cells in droplets

The number of cells per droplet follows a Poisson distribution, which describes the probability of finding a mean number λ of *x* cells per droplet (Howard Shapiro, Pratical Flow Cytometry, 4th edition. Wiley-Liss, 2003). In single-cell ChlP-seq experiment, cell density is adjusted to encapsulate λ = 0.1 cells in 45pl droplets, resulting in 90.5% of empty droplets, 9% of droplets containing one single cell, 0.5% containing two cells and 0.015% containing more than two cells. Real-time monitoring of the compartmentalization of cells in droplets is performed by pre-labelling the cells with Calcein AM, a non-fluorescent derivate of calcein. Upon entering into the cells, the acetomethoxy group (AM) gets cleaved by intracellular esterase and releases a strong green fluorescence (excitation/emission: 495/515nm). The fluorescence is acquired as the droplets crossed the laser beam at the detection point, allowing counting the number of cells encapsulated.

### Droplets collection

Droplets are collected in a collection tube on ice until the end of the encapsulation (10min to 20min depending on the number of starting cells). After encapsulation, drops are incubated at 37°C for MNase digestion.

### MNase calibration in droplets

At the end of the encapsulation, droplets are incubated off-chip for single-cell chromatin fragmentation. Cells are lysed in droplets, making their nuclear DNA accessible for MNase enzyme. The kinetic of the digestion is particularly important to yield preferentially mono-nucleosomes, which are retained in the droplet. The ideal incubation time is defined as the time necessary to get 100% of nuclear DNA fragmented into mono-nucleosomes. The digestion conditions including lysis buffer composition, MNase concentration and incubation time are precisely calibrated for each sample by performing a time-course study. The calibration is carried out as follows: 45pl droplets containing cells, lysis buffer and MNase are produced, collected in a collection tube and placed at 37°C for different incubation time. At each time point, a fraction of droplets is broken and MNase immediately inactivated by addition of EGTA (see Figure 3). DNA fragments are then purified and analyzed by electrophoresis. The choice of the incubation time is a balance between having the highest proportion of mono-nucleosomes but, in the same time, preventing nucleosomal DNA to be over-digested. Indeed, it was hypothesized that DNA protruding from the nucleosome should be long enough to enable an efficient ligation of the barcodes in the subsequent steps of the procedure.

### Controlling MNase activity in droplets

MNase activity in droplets is controlled by collecting the droplets on ice upon cell encapsulation (see Figure 2). Indeed, the time point t = 0min in Figure 2, which corresponded to a fraction of droplets taken at the end of the droplets production but just before incubation, indicates that nuclear DNA is not digested yet by MNase. This evidence confirms that chromatin digestion does not occur upon droplet production but starts immediately with incubation at 37°C (see Figure 2).

Placing the droplets on ice after incubation and upon re-injection in the fusion device may "pause" MNase activity and limit cell-to-cell variation in chromatin digestion. For this purpose, two droplet fractions are taken after 12min MNase incubation: one fraction is immediately processed to control for digestion, while the second fraction is stored beforehand for 1h on ice, then processed similarly. As expected, the time points t = 12min and t = 12min + 1h ice on Figure 2 confirm that MNase is no longer active in the fraction stored on ice (as compared with t = 20min time point). Consequently, storing droplets on ice "pauses" MNase activity, thus preventing again variation in chromatin digestion between droplets

### DNA barcoding strategy

DNA barcodes are grafted to the hydrogel beads via a streptavidin-biotin linkage and a photo-cleavable moiety enabling release from the beads when exposed to UV light (Klein et al. 2015, Cell 161(5):1187-1201). The synthesis of barcodes consists in distributing the beads in a microtiter well plate containing ligation reagents and 96 combinations of a 20 bp oligonucleotide (later referred to as Index 1). Index 1 are ligated to the beads and the latter are pooled before being distributed again in a second microtiter plate containing 96 new combinations of a 20 bp oligo (later referred to as Index 2). By repeating 3 times this split-pool method, a library of 96³ possible barcode combinations is easily generated (i.e. 884,736 combinations).

### Quality controls of barcoded hydrogel beads

Barcoded beads are one of the core reagents of the scChlP-seq technology, their quality have been systematically controlled to ensure that cell-to-cell variations originate from true biological differences in their histone modification patterns rather than technical artefacts.

Tapestation profile of DNA barcodes released from the beads revealed that >75% were full length (larger peak 146 bp), as well as the presence of intermediates that failed to be completed (Figure 5). In average, the number of full-length barcodes is estimated to 5x10⁷ copies per barcoded hydrogel bead.

To validate the release of barcodes from the hydrogel beads, DNA probes are hybridized to the barcodes onto the beads. The latter are then encapsulated in 100pl droplets and collected off-chip as in a scChlP-seq experiment. Part of the droplets are re-injected as a single file into a micrometric chamber as reported by Eyer (Eyer et al. 2017, Nature Biotechnology 35(10):977-982) and imaged the beads by epifluorescence microscopy while the fluorescent barcodes are still bound on the beads. As expected, the fluorescence is localized on the beads (see Figure 5). A second fraction of beads-containing droplets is exposed to UV light to initiate barcodes release. As described above, epifluorescence microscopy of bead-containing droplets after photo-cleavage reveals a uniform distribution of the fluorescence in the droplets, suggesting complete barcode release (see Figure 5). Lastly, single-bead sequencing is performed on every new batch of barcoded beads. Beads are isolated by limiting dilution in a 384-well plate. Only wells containing one bead are selected by imaging for amplification and sequencing of the barcodes. The analysis of the sequencing data shows the fraction of the first two most abundant barcodes of 16 beads. Hundred thousand of different barcodes are identified per bead but, in average, the most abundant barcode represents 97.7% of the sequencing reads. The second most abundant barcode takes on average as few as 0.17% of the reads, suggesting that all the other identified barcodes are negligible (see Figure 5).

### Barcode design

Barcodes are bound to the beads via streptavidin-biotin linkages, which are in turn separated from the 5'-end of the oligonucleotide by photo-cleavable entities. The latter are composed of the photo-cleavable group as well as an alkyl spacer that minimize steric interactions (the all entity is referred to as PC-linker, see Figure 4). The first biotinylated and PC-linker oligonucleotide is common to all barcodes and comprises a T7 promoter sequence and an Illumina sequencing adaptor (SBS12 sequence). The T7 promoter sequence serves as a recognition site for the T7 RNA polymerase to initiate the linear amplification of enriched barcoded nucleosomes post-immunoprecipitation in an *in-vitro* transcription reaction (IVT). This amplification strategy is widely adopted for unbiased, sensitive and reproducible amplification of cDNAs post-reverse transcription in single-cell RNA-seq protocols (Hashimshony et al. 2012, Cell Reports 2(3):666-673). In a second step, the Illumina sequencing adaptor serves as PCR handle to complete the preparation of the sequencing library. Also, this adaptor is necessary for the Next Generation Sequencing of the samples as the primer that initiates Read #2 and the reading of the barcode sequence. The beads grafted with this first common oligonucleotide are then used for barcode synthesis by successive ligation of the 3 indexes. Unfortunately, the analysis of a first single-cell ChlP-seq dataset reveals that only few reads (^{∼}38%) have a complete and correct barcode structure.

An optimized barcode structure allowing the digestion of barcode concatemers as well as reducing the ligation of non-full-length barcodes is depicted in Figure 3. Barcodes are framed with one half of Pacl restriction site, which is only reconstructed in case of formation of concatemers. Those are digested after the immunoprecipitation but before the linear amplification to clean-up the library. Barcodes photo-cleaved side is modified with the incorporation of a 3' C3-spacer. This modification introduces a spacer arm at the 3'-hydroxyl group of the 3' base and blocks the ligation. With the addition of the spacer group, the direction of the ligation is enforced from the 3'-end of the barcodes to the nucleosomes. Non-full-length barcodes are completed with a "block" oligonucleotide sequence comprising a 3' C3-spacer and a 5' inverted dideoxy-T base. Again, both modifications aim at limiting unwanted ligation events.

### Encapsulation of barcoded beads in droplets

Loading discrete objects such as hydrogel beads into droplets can be estimated by a Poisson distribution. In the same way as the encapsulation of the cells, the loading of the beads is monitored in real time as the droplets crossed the laser beam at the detection point. In single-cell ChlP-seq experiment, it is typically achieved between 65% to 75% of droplets that contain a barcoded hydrogel bead.

### Merging nucleosomes-containing droplets with barcodes-containing droplets

Cells and DNA barcodes are separately encapsulated to prevent barcodes digestion by MNase. To index chromatin at the single-cell level, DNA barcodes have to be delivered in a second step into nucleosomes-containing droplets. This is achieved by active fusion of the two droplets populations in a dedicated microfluidic device using a triggered electric field.

Droplets from the "cell emulsion" and droplets from the "barcode emulsion" are re-injected as a single-file in the microfluidic fusion device. Achieving proper electro-coalescence requires one-to-one pairing of the droplets from the two emulsions. Hydrodynamic forces enable the faster smaller 45pl droplets ("cell emulsion") to catch up and come in contact with the 100pl droplets ("barcode emulsion"), as contact is necessary for the two droplets to fuse (Mazutis et al. 2009, Lab on a Chip 9(18):2665). Similarly to droplet productions, fluorescence intensity of fused droplets is acquired as they crossed the laser beam at the detection point (see Figure 1).

### Reconstructing cell type-specific chromatin states from single-cell ChIP-seq profiles

Human T lymphocytes and human B lymphocytes are separately encapsulated and indexed with two distinct sets of barcodes as shown in Figure 7. After nucleosomes barcoding in droplets, indexed chromatin from the two cell types are combined, performed chromatin immuno-precipitation and sequenced the library

Introduction of bias related to the immuno-precipitation or preparation of the sequencing libraries (batch effect) is avoided by combining indexed chromatin. Each sequencing read would carry a double information: (1) the single-cell barcode sequence, which assigns the read to its cell of origin; (2) the "cell-type specific sequence", which assigns the read to one cell type (either B or T lymphocyte).

To confirm that barcodes were unique to a single cell, it has been performed an experiment with a mixture of mouse and human cell lines, which showed that 97% of the barcodes were unambiguously assigned to a single species, consistent with the percentage of occupied droplets containing single cells (95%) as shown in Figure 13.

It was validated the efficiency and accuracy of the scChlP-seq procedure to recapitulate cell identity from the single-cell distribution of H3K4me3 and H3K27me3 modifications. Human Ramos (B cell) and Jurkat (T cell) were processed separately (as in Fig. 1a-b) using two independent sets of barcoded adaptors and, after ligation of the adaptors in droplets, the barcoded nucleosomes were pooled and immuno-precipitated. For H3K4me3 and H3K27me3 histone marks, it was achieved an average coverage of 1,630 and 1,633 unique reads per cell, respectively, and a high correlation across technical and biological replicates (Fig. 14a-c, r = 0.96 and 0.98 with p < 10-15 respectively).

For both single-cell chromatin profiling experiments, it was identified by consensus clustering two stable clusters corresponding to each cell line (Fig. 15a and Fig. 16a), matching cell identity with a specificity of over 99.7% and 99.5% respectively for H3K4me3 and H3K27me3 profiles. Aggregated single-cell profiles recapitulated the bulk ChlP-seq profiles with high accuracy (Fig. 15b-c, r = 0.93 and 0.97 with p < 10-15 for H3K4me3 and H3K27me3 respectively, Fig. 14f). It was identified through differential analysis permissive and repressive chromatin features specific to Ramos and Jurkat cells (Fig. 16b). Focusing on H3K4me3, which accumulates around transcription start sites, we identified concordant lineage-specific sets of genes as being enriched among chromatin features specific to each cell line (Fig. 16c). These results confirmed that the scChlP-seq procedure is a robust method to detect chromatin landscapes at the single-cell level, to classify single cells with a high accuracy according to their chromatin state and to identify discriminating chromatin features between cell populations.

### Deconvolution of single-cell barcodes and cell type-specific sequences

Barcodes were extracted from Reads #2 by first searching for the constant 4 bp linkers found between the 20-mer indices of the barcode allowing up to 1 mismatch in each linker. If the correct linkers were identified, the three interspersed 20-mer indices were extracted and concatenated together to form a 60 bp non-redundant barcode sequence. A library of all 884,736 combinations of the 3 sets of 96 indices (96³) was used to map barcode sequences using the sensitive read mapper Cushaw3. Each set of indices is error-correcting because it takes more than an edit-distance of 3 to convert one index into another. it was therefore set a total mismatch threshold of 3 across the entire barcode, with two or less per index to avoid mis-assigning sequences to the wrong barcode Id. In a second, slower step, sequences that could not be mapped to the Cushaw3 index-library were split into their individual indices and each index compared against the set of 96 possible indices, allowing up to 2 mismatches in each individual index. Any sequences not assigned to a barcode Id by these two steps were discarded.

### Single-cell ChIP-seq data analysis

Sequencing data were analyzed with Python (v2.7.12) and R (v3.3.3) using the reference genome hg38 (GenBank assembly accession: GCA_000001405.15)

### De-multiplexing cellular barcodes - Alignment, filtering & normalization

Reads #1 were aligned to mouse mm10 and human hg38 reference genomes using bowtie (v1.2.2) by keeping only reads having no more than one reportable alignments and 2 mismatches.

Raw reads are distributed according to a bimodal distribution, the lower peak most probably corresponding to droplets with barcoded beads but without cells (Rotem, A. et al. Nat Biotechnol; 2015, 33:1165-1172), and the right peak corresponding to droplets with cell with bead (Figure 6a); thereby setting a read count cut-off to define barcodes associated to a cell. For subsequent analysis, the inventors kept barcodes with a unique (post PCR duplicate removal) read count above this cut-off. To remove PCR duplicates, for each barcode (i.e cell), all the reads falling in the same 150 bp window were stacked into one as reads possibly originating from PCR duplicates or from the same nucleosome. The inventors generated coverage matrix and metrics from these de-duplicated reads, referred to as 'unique reads' in the text.

For each cell, reads were binned in non-overlapping 50 kb for H3K27me3, known to accumulate over broad genomic regions, and 5 kb genomic bins for H3K4me3, known to accumulate in narrow peaks around transcription start sites, spanning the genome to generate a n x m coverage matrix with n barcodes and m genomic bins. The inventors combined coverage matrices for each of the four analyses from the following samples: (i) Ramos and Jurkat, (ii) mouse cells from HBCx95 and HBCx-95-CapaR, (iii) human cells from HBCx95 and HBCx95-CapaR, and (iv) human cells from HBCx-22 and HBCx-22-TamR.

The inventors first removed cells with a total number of uniquely mapped reads within the upper percentile, considered as outliers, and filtered out genomic regions not represented in at least 1% of all cells. By PCA analysis, the inventors could group cells independently of coverage only if cells had at least 1,600 unique reads per cell. For all subsequent analyses, the inventors excluded cells with lower coverage. Coverage matrices were then normalized by dividing counts by the total number of reads per cell and multiplying by the average number of reads across all cells.

### Unsupervised clustering of single-cell ChlP-seq profiles

Normalized matrices were reduced by principal component analysis (n = 50 first components selected for further analysis). To improve the stability of the clustering approaches, the inventors further limited the analysis to cells displaying a Pearson's pairwise correlation score above a threshold t with at least 1% of cells. Threshold t was defined as the upper percentile of Pearson's pairwise correlation scores for a randomized dataset.

The inventors used consensus clustering, Bioconductor ConsensusClusterPlus package (Wilkerson, M. D. & Hayes, D. N. 2010. Bioinformatics 26, 1572-1573), to examine the stability of the clusters and compute item consensus score for each cell. The inventors established consensus partitions of the data set in k clusters (for k = 2, 3, ...), on the basis of 1,000 resampling iterations (80% of cells) of hierarchical clustering, with Pearson's dissimilarity as the distance metric and Ward's method for linkage analysis. The optimal number of clusters (k) was chosen to maximize intra-cluster correlation score. Clustering results were visualized with t-SNE plots (Van der Maaten, L. & Hinton, G. 2008. J Mach Learn Res 9, 2579-2605). To visualize chromatin profiles of subpopulations, the inventors aggregated reads of single-cells within each cluster and created enrichment profiles using the R package Sushi (Phanstiel, D. H. et al. 2014. Bioinformatics. 30, 2808-2810).

### Differential analysis of single-cell ChIP-seq profiles

To identify differentially enriched regions across single-cells for a given cluster, the inventors performed a non-parametric Wilcoxon rank sum test comparing normalized counts from individual cells from one cluster versus all other cells. The inventors testes for the null hypothesis that the distribution of normalized counts from the two compared groups have the same median, with a confidence interval 0.95. The inventors limited analysis to the windows selected for unsupervised analysis above.

P-values were corrected for multiple testing using Benjamini-Hochberg procedure (Benjamini, Y. & Hochberg, Y. 1995. J R Stat Soc 57, 289-300). Genomic regions were considered as 'enriched' or 'depleted' for H3K27me3 or H3K4me3 if adjusted p-values, 'q-values', were lower than 0.01 and the absolute log2 fold change greater than 1.

### scRNA-seq comparison.

For H3K27me3 scChlP-seq analysis, the inventors used peak annotation from bulk ChlP-seq datasets to further annotate 50kbp windows and corresponding genes: for each window, the inventors kept for subsequent analyses (gene annotation and scRNA-seq comparison), genes with a transcription start site (TSS) overlapped by a peak in any condition, using bedtools (v2.17)50 and the reference annotation of the human transcriptome Gencode_hg38_v26, limited to protein_coding, antisense and IncRNA genes.

### Bulk ChlP-seq

ChIP experiments were performed as described previously in Vallot, C. et al. 2015. Cell Stem Cell 16, 533-546, on 106 cells from cell suspensions obtained above from HBCx-22, HBCx-22-TamR, HBCx-95 and HBCx-95-CapaR using anti-H3K27me3 antibody (Cell Signaling Technology, # 9733). 2 ng of immune-precipitated and input DNA were used to prepare sequencing libraries using the Ovation Ultralow Library System V2 (Nugene) according to the manufacturer's instructions. Bulk ChlP-seq libraries were sequenced on an Illumina HiSeq 2500 in Rapid run mode SE50.

### Bulk ChIP-seq data analysis

Reads were aligned to mouse mm10 and human hg38 reference genomes using bowtie (v1.2.2) and the tool bamcmp was used to separate human from mouse sequences. Subsequent analysis was performed as explained previously. Only uniquely mapping reads were kept for the analysis, in addition PCR duplicates were removed using Picard Tools (https://broadinstitute.github.io/picard/). Data were binned in 5 kb (H3K4me3) or 50 kb (H3K27me3) consecutive genomic windows. For each window, log 2 RPM were computed, as the logged number of reads per millions of mapped reads.

### Single-cell RNA-seq

Approximately 3,000 cells from each cell suspensions, HBCx-22, HBCx-22-TamR, HBCx-95 and HBCx-95-CapaR, were loaded on a Chromium Single Cell Controller Instrument (10X Genomics) according to the manufacturer's instructions. Samples and libraries were prepared according to the manufacterer's instructions. Libraries were sequenced on Illumina an HiSeq 2500 in Rapid run mode, using paired-end 26 bp - 98 bp sequencing.

### Single-cell RNA-seq data analysis

Single-cell sequencing files were processed using the Cell Ranger Single Cell Software Suite (v1.3.1) to perform quality control, sample de-multiplexing, barcode processing, and single-cell 3' gene counting (http://software.10xgenomics.com/single-cell/overview/welcome) using the UCSC mouse (mm10) and human (hg19) transcriptome and genome with default parameters. 2,728 cells with an average coverage of 30,166 reads/cell (1,564 human and 1,191 mouse cells) for HBCx-22, 1,746 cells with an average coverage of 41,166 reads/cell (753 human and 1,013 mouse cells) for HBCx-22-TamR, 1,184 cells with an average coverage of 160,583 reads/cell (545 human and 647 mouse cells) for HBCx-95, 2,087 cells with an average coverage of 38,345 reads/cell (861 human and 1,242 mouse cells) for HBCx-95-CapaR were analyzed. Further analysis was performed in R (v3.3.3) using custom R scripts. Any cell with more than 10% of mitochondrial UMI counts was filtered out. The inventors only kept cells with a total count of UMI below 100,000 and of detected genes below 6,000 and over 1,000. The inventors then only kept genes with at least 1 transcript in at least 2 cells. Using the R package scater, scRNAseq count matrices were normalized for coverage and transformed by RLE, 'Relative Log Expression' method (McCarthy, D. J et al. Bioinformatics, 2017, 33:1179-1186). Using annotations from the R package ccRemover (Barron, M. et al. Sci Rep, 2016, 6:33892), the inventors removed genes related to cell cycle from subsequent clustering analyses to group cells according to cell identity and not cell-cycle related phenomena. Barnes-hut approximation to t-SNE was then performed on the n = 50 first principal components (PCA) to visualize cells in a two-dimensional space. Clusters were identified using Consensus Clustering as for scChlP-seq analyses above. The inventors identified genes that were differentially expressed between clusters using edgeR GLM statistical models (Robinson, M. D. et al. Bioinformatics, 2010, 26:139-140). For stromal mouse cells, clusters were identified according to the differential expression of hallmark genes.

### Copy number profiles of bulk tumor cells

The R package HMMcopy was used to correct for copy number variation in non-treated versus resistant xenograft models. Reads from bulk input ChlP-seq samples were binned in 0.5 Mb non-overlapping regions spanning the genome. Regions with a deviation to the mean greater than n = 2 standard deviations were removed for analysis.

Here following it is shown an example, wherein the method of the invention is used for the diagnosis and/or prognosis of the of drug resistance by profiling single cell chromatin states in untreated and/or treated cells using a microfluidic system.

It is here shown how a scChlP-seq experiment can interrogate the heterogeneity of chromatin marks within a triple-negative breast tumor model of acquired resistance to chemotherapy.

As used herein, "triple-negative tumor" refers to a breast cancer characterized by being ER-, PR- and HER2-.

For the method according to the invention, after two cycles of treatment of xenograft derivatives from an individual patient, initially responsive to Capecitabine, it was obtained a tumor with acquired resistance to Capecitabine, HBCx-95-CapaR (Fig. 17a). It was profiled the H3K27me3 landscape at single-cell resolution for both sensitive and resistant xenografts, HBCx-95 and HBCx-95-CapaR (Fig. 17b), and it was also performed scRNA-seq to evaluate transcriptional heterogeneity within the same cell suspension (Fig. 18a). Cumulative single-cell chromatin profiles match bulk ChlP-seq profiles (Fig. 17c-d, r = 0.89 with p < 10-15).

It was studied the diversity of chromatin profiles within stromal cells (n= 1,766 mouse cells with average coverage of 3,535 unique reads per cell). Consensus clustering approaches (Fig. 19) show that stromal cells stably grouped in three chromatin-based populations according to H3K27me3 profiling, Chrom_c1, c2 and c3, irrespectively of whether the patient-derived xenograft (PDX) w sensitive or resistant to treatment (Fig. 18b), arguing against a potential batch effect. By comparing chromatin features between groups of cells (Fig. 20), it was identified loci with specific H3K27me3 enrichment for Chrom_c2 and c3 populations (n = 1,581 and n = 1,282 respectively with q-value < 0.01 and |log2FC| > 1), and to a lesser extent for cluster Chrom_c1 (n = 122).

In parallel, scRNA-seq analysis revealed four populations of stromal cells (Fig. 18c and Fig. 21): two groups of cells of fibroblast origin (with specific markers Coll2a1 and Efemp1), endothelial cells (Pecam-1) and macrophage cells (Ms4a7). To further compare the identity of populations inferred from both approaches, particular interest was focused on genes with a transcription start site located within 1 kb of chromatin features specific of Chrom_c1, c2 and c3. Loci specifically devoid of H3K27me3 in cells from Chrom_c2, i.e. permissive for transcription only in Chrom_c2, were significantly enriched in genes involved in epithelial to mesenchymal transition (q-value =2.8x10-3), such as Col4a1 (Fig. 20b), or in apical junction (q-value =9.0x10-2), such as Ptk2 (Fig. 18d); both signatures being characteristic of fibroblast expression programs. Similarly, it was found loci devoid of H3K27me3 specific to Chrom_c3 enriched in genes from immune expression programs (q-value =5.2x10-2, Fig. 20a), such as Lrmp (Fig. 18e). The scRNA-seq data further confirmed the expression pattern of these markers within the PDX samples (right panels Fig. 18d-e). It could not be identified relevant genes associated to the few chromatin marks characteristic of Chrom_c1, either suggesting that these cells were less efficiently captured with the scChlP-seq procedure according to the invention or that this cluster of cells shared chromatin features with both Chrom_c2 and c3. Indeed, half of the cells from this cluster share with immune-like cells H3K27me3 enrichment for Ptk2 (Fig. 18d). Altogether the scChlP-seq approach according to the invention revealed the existence of three H3K27me3 chromatin landscapes within mouse stromal cells, two of which matched transcriptomic signatures identified with scRNA-seq (fibroblast and immune-like signatures).

Next, it was investigated the heterogeneity of chromatin profiles among tumor cells from the same pair of triple-negative breast tumor samples (n = 4,331 cells from HBCx-95 and HBCx-95-CapaR, with average coverage of 5,161 unique reads per cell). Loci affected by copy-number variations, as identified from bulk DNA profiles, to focus on chromatin alterations (Fig. 22a) were removed from the analysis. Based on both chromatin and transcriptomic profiles, cells clustered primarily according to their sensitive or resistant tumor origin (Fig. 23a-c and Fig. 22b-c). While the chromatin profiles of sensitive cells were largely homogeneous, distinct chromatin states within the resistant population were apparent (Fig. 23a), suggesting that heterogeneous populations of resistant cells, with distinct chromatin features, emerged. However, consensus clustering also showed that 3% of cells from the untreated tumor (n = 13 out of 484) robustly classify with resistant cells (Fig. 23d, Consensus Score over 0.9), suggesting that they share common chromatin features. Resistant-like and resistant cells, corresponding to Chrom_c2, display a high number of loci depleted in H3K27me3 compared to sensitive cells from Chrom_c1 (Fig. 23e-f, n = 569 loci with depleted versus 114 with enriched H3K27me3, q-value < 0.01 and |log2FC| > 1, 30% overlapping a transcription start site). Loci specifically devoid of H3K27me3 in cells from Chrom_c2 were enriched in genes targets of the Polycomb complex (Fig. 22d), indicating a demethylation of expected EZH2 targets. It could be only detected transcription within 5% of these loci, either due to the absence of transcription or to insufficient sensitivity of the scRNA-seq procedure. Within these loci, six genes were significantly deregulated according to scRNA-seq, and all in accordance to their H3K27me3 chromatin states (Fig. 23f, Fig. 22e). Interestingly, it was identified IGF2BP3, a gene known to promote resistance to chemotherapy (Fig. 23g) and regions with markers of epithelial-to-mesenchymal transition (COL4A2, HOXD cluster, Fig. 23h-i), which induces resistance to chemotherapy.

In addition, it was profiled a pair of luminal ER+ breast PDXs: HBCx-22, responsive to Tamoxifen and HBCx-22-TamR, a tumor derivative with previously characterized acquired resistance to Tamoxifen. To obtain a high average coverage of 10,228 unique reads per cell, it was limited the number of encapsulated cells (n = 822 tumor cells, Fig. 25a-b). Tumor cells displayed two major chromatin profiles related to their tumor of origin. However, 16% (n = 41 out of 255) of cells within the sensitive tumor shared chromatin features with all resistant cells (Fig. 24a-c and Fig. 25c). Chromatin features characteristic of resistant cells were already found in rare cells from the sensitive tumor, and could have been selected for by Tamoxifen treatment.

Differential analysis of chromatin features revealed that resistant-like and resistant cells *(Chrom_c2)* have predominantly lost H3K27me3 marks compared to sensitive-like cells (Fig. 25d, n = 356 loci with depleted versus 137 with enriched H3K27me3). Loci specifically devoid of H3K27me3 in cells from *Chrom_c2* were enriched in genes targets of the Polycomb complex, and characteristic of basal-like signatures of the mammary epithelium (Fig. 25e). With scRNA-seq, it could be only detected a transcription in 2% of differentially enriched windows, and significant differential expression for 3 genes, all showing transcription activation in a fraction of resistant cells in mirror to their loss of H3K27me3 enrichment: *EGFR,* a gene implicated in resistance to Tamoxifen, *IGFBP3* and *ALCAM* (Fig. 24d, h-i and Fig. 25g).

Parallel scRNA-seq analysis of the same cell suspension revealed several clusters within the resistant and sensitive tumor (Fig. 24e-f, Fig. 25f). While no cells from the sensitive tumor cluster with the resistant cells, it is here shown that cells from the RNA_c6 cluster, originating from the sensitive tumor (corresponding to 17%, 211 out of 1,275 cells),display activation of the pathways characteristic of resistant tumor cells, among which basal-like gene signatures and signature of epithelial to mesenchymal transition (Fig. 24g). These observations independently confirm that non-genetic features common to resistant cells, either at the transcriptomic or chromatin-level, are found in cells from the sensitive tumor. Both single-cell measurements point towards the activation of basal-like gene signatures, but through different sets of genes.

Profiling histone modifications at the single-cell level with high coverage, up to 10,000 loci in average per cell, was instrumental to reveal the presence of relatively rare chromatin states within tumor samples. The single-cell chromatin profiling appears as a unique tool to probe the role of heterogeneity and dynamics of chromatin states within any complex biological system: in addition to cancer it can be applied to other diseases and healthy systems, notably to study cellular differentiation and development for patient stratification.

The method according to the invention can be used to unveil that rare cells with chromatin features characteristic of resistant cancer cells exist *before* treatment and could be selected for by cancer therapy. As mentioned already, sspontaneous heterogeneity of chromatin states in untreated cells may be a key molecular component in the acquisition of drug-resistance, regardless of the mechanism of action of the cancer treatment: here Tamoxifen, targeting the estrogen receptor, and Capecitabine, a classical chemotherapy inhibiting the synthesis of thymidine monophosphate.

The method according to the invention unveil perspectives for novel therapeutic strategies both in luminal and triple-negative breast cancer to counteract resistance. For example, preventing loss of repressive chromatin marks such as H3K27me3, as observed in resistant cells, by combining treatment with Capecitabine and Tamoxifen with drugs such as demethylase inhibitors could be a strategy to consider minimizing resistance. In addition, loci depleted for the transcriptional repressive mark H3K27me3 in resistant-like cells, included genes known to promote resistance to chemotherapy or targeted therapy, highlighting the potential to discover new drug targets and biomarkers for patient stratification. Interestingly, both models share the H3K27me3-demethylation, and transcription activation of a gene of the *IGFBP* family, in the IGF signaling pathway, which plays a key role in breast cancer and drug resistance.

In the example, the model of acquired resistance to Tamoxifen, scRNA-seq and scChlP-seq independently revealed the existence of a fraction of cells within the sensitive tumor with non-genetic features common to resistant cells. The two approaches did not reveal the same subset of genes, but revealed common gene signatures characteristic of a switch from luminal to basal cell identity. Such differences could highlight the poor sensitivity of current single-cell RNA-seq approaches or reveal that a fraction of genes is poised for transcription, but not transcribed. Loss of repressive chromatin marks such as H3K27me3 could change the chromatin to a permissive state and could correspond to a priming event preceding changes in transcription.

## Claims

1. A method for the diagnosis and/or prognosis of drug resistance wherein single cell chromatin states are profiled in cells obtained from a subject by using a microfluidic system, the method comprising the steps of:
a. providing at least a droplet of first type, wherein said droplet of first type comprises
i. a biological element,
ii. a lysis buffer, and
iii. a nuclease,
b. collecting said droplet of first type under conditions that temporarily inactivate said nuclease,
c. incubating said droplet of first type, thereby reactivating said nuclease,
d. providing at least a droplet of second type, wherein said droplet of second type comprises a nucleic acid sequence,
e. merging said droplets of first and second type, thereby generating a droplet of third type,
f. incubating said droplet of third type, thereby linking said nucleic acid sequence to one or more genomic region(s) of interest,
g. sequencing said one or more genomic region(s) of interest.

2. The method according to claim 1, wherein the one or more genomic region of interest is a modified genomic region.

3. The method according to any of the preceding claims, wherein the modified genomic region comprises a nucleic acid sequence and/or a protein complex associated with a nucleic acid sequence.

4. The method according to any of the preceding claims, wherein the modified genomic region comprises a post-translational modification selected from the group comprising acetylation, amidation, deamidation, carboxylation, disulfide bond, formylation, glycosylation, hydroxylation, methylation, myristoylation, nitrosylation, phosphorylation, prenylation, ribosylation, sulphation, sumoylation, ubiquitination and derivatives thereof, preferably a methylation.

5. The method according to any of the preceding claims, wherein the cells derive from a subject in a diseased state and/or suspected to be in a diseased state or in a healthy subject.

6. The method according to any of the preceding claims, wherein the subject is in a diseased state, and the diseased state comprises cancer, infectious disease, auto-immune disease, metabolic disease, inflammation disease, genetic and non-genetic diseases.

7. The method according to claim 6, wherein the diseased state is breast cancer.

8. The method according to any of the preceding claims, wherein the cells are untreated and/or treated.

9. The method according to claim 8, wherein the treated cells are treated with a therapeutic agent, or a chemotherapeutic agent, or a chemical drug, or biologic drugs.

10. The method according to claim 8, wherein the treated cells are treated with Tamoxifen and Capecitabine.

11. The method according to any of the preceding claims, wherein the single cell chromatin state has lost chromatin marks for genes that promote drug resistance.

12. The method according to claim 11, wherein the chromatin marks are histone modifications H3K4me3 and H3K27me3.
